# EUROPEAN PATENT SPECIFICATION

(11) **EP 2 245 455 B1**
(45) Date of publication and mention of the grant of the patent: **09.07.2014**
(21) Application number: 09702668.6
(22) Date of filing: 16.01.2009
(51) Int. Cl.: G01N 33/50, A01K 67/027

(54) **METHOD FOR DISTINGUISHING SECRETORY GRANULES OF DIFFERENT AGES**
VERFAHREN ZUR UNTERSCHEIDUNG SEKRETORISCHER GRANULA UNTERSCHIEDLICHEN ALTERS
PROCÉDÉ POUR DISTINGUER DES GRANULES DE SÉCRÉTION DE DIFFÉRENTS ÂGES

(30) Priority: 17.01.2008 EP 08000875; 25.02.2008 EP 08003393
(43) Date of publication of application: 03.11.2010
(73) Proprietor: Technische Universität Dresden Medizinische Fakultät Carl Gustav Carus, 01307 Dresden (DE)
(72) Inventor: SOLIMENA, Michele, 01326 Dresden (DE); IVANOVA, Anna, 01097 Dresden (DE)
(74) Representative: Vossius & Partner
(86) International application number: PCT/EP2009/000265
(87) International publication number: WO 2009/090090

(56) References cited:
- WIEGAND U K ET AL: "Red, yellow, green go! A novel tool for microscopic segregation of secretory vesicle pools according to their age." BIOCHEMICAL SOCIETY TRANSACTIONS, vol. 31, no. 4, August 2003 (2003-08), pages 851-856, XP002479969 ISSN: 0300-5127
- RUDOLF R ET AL: "DYNAMICS OF IMMATURE SECRETORY GRANULSES: ROLE OF CYTOSKELETAL ELEMENTS DURING TRANSPORT, CORTICAL RESTRICTION, AND F-ACTIN-DEPENDENT TETHERING" MOLECULAR BIOLOGY OF THE CELL, BETHESDA, MD, US, vol. 12, no. 5, 1 May 2001 (2001-05-01), pages 1353-1365, XP001155802 ISSN: 1059-1524
- NORDMANN J J ET AL: "METHOD FOR QUANTITATING THE MOLECULAR CONTENT OF A SUB CELLULAR ORGANELLE HORMONE AND NEUROPHYSIN CONTENT OF NEWLY FORMED AND AGED NEURO SECRETORY GRANULES" PROCEEDINGS OF THE NATIONAL ACADEMY OF SCIENCES OF THE UNITED STATES OF AMERICA, vol. 81, no. 1, 1984, pages 180-184, XP002479970 ISSN: 0027-8424
- GAUTIER ET AL: "An Engineered Protein Tag for Multiprotein Labeling in Living Cells" CHEMISTRY AND BIOLOGY, CURRENT BIOLOGY, LONDON, GB, vol. 15, no. 2, 22 February 2008 (2008-02-22), pages 128-136, XP022489104 ISSN: 1074-5521
- BOHME ET AL: "Tracking of human Y receptors in living cells-A fluorescence approach", PEPTIDES, ELSEVIER, AMSTERDAM, vol. 28, no. 2, 31 January 2007 (2007-01-31), pages 226-234, XP005738489, ISSN: 0196-9781, DOI: 10.1016/J.PEPTIDES.2006.08.042
- BIE AND PAN, [Online] 2007, Molecular Pain, vol. 3, 2007, p. 37 Retrieved from the Internet: <URL:http://www.molecularpain.com/content/3 /1/37/figure/F1?highres=y> [retrieved on 2012-08-16]
- ROBERTI MARIA J ET AL: "Fluorescence imaging of amyloid formation in living cells by a functional, tetracysteine-tagged alpha-synuclein", NATURE METHODS, vol. 4, no. 4, April 2007 (2007-04), pages 345-351, ISSN: 1548-7091
- LEE HE-JIN ET AL: "Intravesicular localization and exocytosis of alpha-synuclein and its aggregates", JOURNAL OF NEUROSCIENCE, vol. 25, no. 25, June 2005 (2005-06), pages 6016-6024, ISSN: 0270-6474
- SUCKALE J ET AL: "The insulin secretory granule as a signaling hub", TRENDS IN ENDOCRINOLOGY AND METABOLISM, ELSEVIER SCIENCE PUBLISHING, NEW YORK, NY, US, vol. 21, no. 10, 1 October 2010 (2010-10-01), pages 599-609, XP027353784, ISSN: 1043-2760 [retrieved on 2010-07-06]
- MAREK KURT W ET AL: "Transgenically encoded protein photoinactivation (FlAsH-FALI): Acute inactivation of synaptotagmin I.", NEURON, vol. 36, no. 5, 5 December 2002 (2002-12-05), pages 805-813, ISSN: 0896-6273
- ADAMS S R ET AL: "New Biarsenical Ligands and Tetracysteine Motifs for Protein Labeling in Vitro and in Vivo: Synthesis and Biological Applications", JOURNAL OF THE AMERICAN CHEMICAL SOCIETY, ACS PUBLICATIONS, US, vol. 124, no. 21, 1 January 2002 (2002-01-01), pages 6063-6076, XP002468937, ISSN: 0002-7863, DOI: 10.1021/JA017687N
- WESSELS ET AL: "Impact of live cell imaging on coated vesicle research", SEMINARS IN CELL AND DEVELOPMENTAL BIOLOGY, ACADEMIC PRESS, GB, vol. 18, no. 4, 1 August 2007 (2007-08-01) , pages 412-423, XP022235238, ISSN: 1084-9521, DOI: 10.1016/J.SEMCDB.2007.07.002
- GENG XUEHUI ET AL: "alpha-Synuclein binds the K-ATP channel at insulin-secretory granules and inhibits insulin secretion", AMERICAN JOURNAL OF PHYSIOLOGY - ENDOCRINOLOGY AND METABOLISM, vol. 300, no. 2, February 2011 (2011-02), pages E276-E286, ISSN: 0193-1849(print)
- GAIETTA G ET AL: "Multicolor and electron microscopic imaging of connexin trafficking", SCIENCE, AMERICAN ASSOCIATION FOR THE ADVANCEMENT OF SCIENCE, WASHINGTON, DC; US, vol. 296, no. 5567, 19 April 2002 (2002-04-19), pages 503-507, XP002403901, ISSN: 0036-8075, DOI: 10.1126/SCIENCE.1068793
- PERRAIS DAVID ET AL: "Recapture after exocytosis causes differential retention of protein in granules of bovine chromaffin cells", JOURNAL OF PHYSIOLOGY (OXFORD), vol. 560, no. 2, 15 October 2004 (2004-10-15), pages 413-428, ISSN: 0022-3751

## Description

The present invention relates to an in vitro method of detecting secretory granules (SGs) of different ages in a cell by differentially labelling said SGs according to their age as characterised in the appended claims comprising (a) contacting a cell capable of forming SGs and expressing fusion protein comprising (i) a (poly)peptide specific for SGs and (ii) a (poly)peptide having a binding site common for at least three different substances A, B and C covalently binding thereto, wherein said substances are capable of penetrating the cell membrane and wherein at least substances A and C are detectable by different means with a substance A targeting said binding site (b) saturating said binding sites by contacting the cell with a substance B which blocks the binding sites which were not bound by substance A, (c) allowing that unbound substance B is removed from the cell or removing unbound substance B from the cell, (d) contacting the cell with a substance C targeting said binding site on the fusion protein becoming available for binding after step (b) and (e) detecting for the presence of both detectable substances A and C, wherein the simultaneous detection of both substances A and C is indicative of the presence of two populations of SGs having a different age. Further, the present invention relates to an in vitro method of investigating in a cell the effect of a stimulus on secretory granules (SGs) differentially labelled according to their age.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Secretory granules (SGs) are the organelles devoted to the storage of peptide hormones in peptide-secreting endocrine cells. Chemical stimuli induce the fusion of SGs with the plasma membrane and the release of their content in the extracellular space. Like most secretory proteins, peptide hormones are co-translationally translocated into the lumen of the rough endoplasmic reticulum, then they are carried to the Golgi complex and finally they are sorted into nascent SGs. Along this route peptide hormones can undergo multiple post-translational modifications, including proteolytic cleavage and glycosylation. Thus, the generation of SGs is a slow process, which requires more than 30 minutes. As sustained stimulation leads to a progressive depletion of SGs, cells must quickly activate transcriptional and post-transcriptional mechanisms to renew their pool of these organelles.

Since their discovery by electron microscopy more than fifty years ago secretory granules (SGs, also known as large dense core vesicles) of neuroendocrine cells have attracted the attention of cell biologists. This is because they are the predominant neuroendocrine organelles, have an electron dense content, and are responsible for the regulated release of peptide hormones and neuropeptides. SGs are membrane-enclosed spherical organelles with the diameter of several hundred nm. When SGs originate from the trans-Golgi network (TGN) they are still immature. The progressive processing and packaging of peptide cargoes lead to the increasing condensation of their electrodense core and their conversion into mature SGs (Glombik and Gerdes, 2000). SGs are typically distinguished based on their morphological appearance and high immunoreactivity for cell type specific peptide hormones and other more widespread cargoes, including chromogranin A (CgA), B (CgB), and secretogranins II-VI (Taupenot et al., 2003), as well as prohormone processing enzymes, including prohormone convertases 1/3 (PC 1/3) and 2 (PC2) and carboxypeptidase E/H (CPE) (Steiner, 1998). As recently proposed (Meldolesi et al., 2004), additional minimal criteria for recognizing bona fide mature SGs should also comprise their prolonged storage in resting cells; the inclusion of specific v-SNAREs proteins that are essential for regulated fusion of SGs with the plasma membrane, and the lack of endosomal or lysosomal markers. Notwithstanding their distinctive properties from other organelles, SGs are a heterogeneous vesicular population even within a single cell type. For instance, they can differ in size and kinetics of content release (Perrais et al., 2004; Grabner et al., 2005; Michael et al., 2006). Some of these differences may depend on SG aging, since content condensation may progressively reduce granule size and the speed with which cargoes dissolve in the extracellular space. Aging can also affect the probability of SGs to undergo exocytosis, with newly generated SGs being preferentially released (Duncan et al., 2003; Solimena and Gerdes, 2003). Once a mature SG is formed, it retains its identity throughout the exoendocytic cycle. Following exocytosis, in particular, SG membranes are recaptured mainly intact (Taraska et al., 2003), although some transmembrane proteins like synaptotagmin-1 and the V-SNARE VAMP2/synaptobrevin2 can diffuse from sites of exocytosis (Tsuboi et al., 2004), while the cytosolic tail of ICA512/IA-2 can be shaved (Borgonovo et al., 2006).

Duncan et al. (2003) found that granules are spatially and functionally segregated according to their age. However, up to now, no feasible, reliable and rapid method for distinguishing SGs of different ages is known. Such a method would be highly advantageous e.g. to further examine SG function and to provide methods to screen for compounds potentially useful for treating diseases associated with defects in SG synthesis and/or secretion.

Roberti et al. 2007 (Nature Methods, 4:345-351) describes fluorescent imaging of the dynamic processes leading to amyloid deposits in living cells employing a fusion protein of α-synuclein and a tetracysteine-tag as target for fluorogenic biarsenical compounds.

Adams et al. 2002 (Am. Chem. Soc., 124:6063-6076) describes biarsenical ligands and tetracysteine motifs for protein labelling *in vitro* and *in vivo* as well as a host, namely HeLa cells, expressing a fusion protein of synaptobrevin and a tetracysteine tag.

The solution to this technical problem is achieved by providing the embodiments characterized in the claims.

Accordingly, in a first aspect, the present invention relates to an in vitro method of detecting secretory granules (SGs) of different ages in a cell by differentially labelling said SGs according to their age as characterised in the appended claims comprising: (a) contacting a cell capable of forming SGs and expressing a fusion protein comprising (i) a (poly)peptide specific for SGs and (ii) a (poly)peptide having a binding site common for at least three different substances A, B and C covalently binding thereto, wherein said substances are capable of penetrating the cell membrane and wherein at least substances A and C are detectable by different means with a substance A targeting said binding site (b) saturating said binding sites by contacting the cell with a substance B which blocks the binding sites which were not bound by substance A, (c) allowing that unbound substance B is removed from the cell or removing unbound substance B from the cell, (d) contacting the cell with a substance C targeting said binding site on the fusion protein becoming available for binding after step (b) and (e) detecting for the presence of both detectable substances A and C, wherein the simultaneous detection of both substances A and C is indicative of the presence of two populations of SGs having a different age.

The term "differentially labeling said SGs according to their age" as used in the present invention means that the populations of SGs having a different age can be labeled depending on their age. The principle of the method of the invention is to label SGs present in a cell and, after a certain time, to label newly formed SGs differently.

The term "contacting" in accordance with the present invention denotes the application of a substance to the cell. "Contacting" comprises directly contacting the cell with the substance as well as transfecting or transforming a cell with a nucleic acid encoding a substance, in this case a (poly)peptide.

Cells capable of forming SGs are neuroendocrine cells such as beta cells of the pancreatic islets, other pancreatic islet cell types, chromaffin cells of the adenal medulla, cells of the pituitary gland, neuroendocrine cells of the grastrointestinal tract and neurons of the central and peripheral nervous system. Neuroendocrine cells are a specialized group of cells that produce peptide hormones and neuropeptides. They package the hormones in vesicles, sometime together with amines and by other specialized signaling molecules derived from amino acids, and secrete these hormones via exocytosis either into the blood stream or in the neuronal surroundings upon stimulation by various agents, e.g. nutrients, hormones and neurotransmitters. The hormones then travel to their target cells and may stimulate, inhibit or maintain at least one function of these cells. The target cells may feed back information to these neuroendocrine cells that regulates further secretion.

The term "specific for SGs" characterizes (poly)peptides or proteins which are specifically occurring in SGs or are specifically enriched in or on SGs.

The term "expressing a fusion protein" relates to the transcription and translation of fusion proteins using appropriate expression control elements that function in the chosen cell. To this end, the nucleic acid molecule encoding the fusion protein according to the invention which is not naturally occurring in the examined cell may be cloned into a suitable expression vector, the composition of which, generally, depends on the expression system. For the present invention, the expression system is eukaryotic, preferably mammalian. A typical mammalian expression vector contains a promoter element, which mediates the initiation of transcription of mRNA, the protein coding sequence, and signals required for the termination of transcription and polyadenylation of the transcript. Additional elements might include enhancers, Kozak's sequences and intervening sequences flanked by donor and acceptor sites for RNA splicing. Highly efficient transcription can be achieved with the early and late promoters from SV40, the long terminal repeats (LTRs) from retroviruses, e.g., RSV, HTLVI, HIVI, and the early promoter of the cytomegalovirus (CMV). However, cellular elements can also be used (e.g., the human actin promoter).
Alternatively, the fusion proteins can be expressed in stable cell lines that contain the gene construct integrated into a chromosome. The co-transfection with a selectable marker such as dhfr, gpt, neomycin, hygromycin allows the identification and isolation of the transfected cells. The transfected nucleic acid can also be amplified in the cell to express large amounts of the encoded (poly)peptide. The dhfr (dihydrofolate reductase) marker is useful to develop cell lines that carry several hundred or even several thousand copies of the gene of interest. Another useful selection marker is the enzyme glutamine synthase (GS) (Murphy et al. 1991, Biochem J. 227:277-279; Bebbington et al. 1992, Bio/Technology 10:169-175). Using these markers, the mammalian cells are grown in selective medium and the cells with the highest resistance are selected. Appropriate culture media and conditions for the above-described host cells are known in the art.

The term "(poly)peptide" as used herein describes a group of molecules which comprises the group of peptides, consisting of up to 30 amino acids, as well as the group of polypeptides, consisting of more than 30 amino acids. In the latter case, the term "polypeptide" is interchangeably used with "protein". Also in line with the definition the term "(poly)peptide" may describe fragments of proteins. (Poly)peptides may further form dimers, trimers and higher oligomers, i.e. consisting of more than one (poly)peptide molecule. (Poly)peptide molecules forming such dimers, trimers etc. may be identical or non-identical. The corresponding higher order structures are, consequently, termed homo- or heterodimers, homo- or heterotrimers etc. The terms "polypeptide" and "protein" also refer to naturally modified polypeptides/proteins wherein the modfication is effected e.g. by glycosylation, acetylation, phosphorylation and the like. Such modifications are well known in the art.

The term "binding site common for at least three different substances A, B and C covalently binding thereto" characterizes an area located on a (poly)peptide to which at least three different substances specifically and covalently bind. In this regard the binding sites may be identical or overlapping to the extent that as soon as one substance A, B, or C is bound, none of the remaining substances can bind any more. In context with the present invention, binding has to be covalent to avoid competition of substances A, B or C with each other for the common binding site on the fusion protein. If competition was present, the result of the method of the invention would in part depend on the binding affinities of each substance and would thus not be reliable. Furthermore, binding of the three substances specifically occurs on said (poly)peptide and not on any other (poly)peptide or other non-proteinaceous structure present in the cell in which the method is carried out.

A substance which specifically and covalently binds to a (poly)peptide is useful in the present invention if it is possible to modify said substance by alternative coupling of one of a repertoire of at least two detectable, preferably fluorescent compounds thereto while retaining its binding specificity to the (poly)peptide. This results in at least two different detectable substances. The third substance necessary for the method of the present invention would then be the initial substance prior to the modifications effected. In this regard, target binding sites suitable in the present invention can be any binding site for a substance on a (poly)peptide as long as it is specific for said (poly)peptide. Examples for such (poly)peptides comprise those having a binding site for a substance which does not bind to any other proteinaceous or non-proteinaceous structure in the examined cell. Binding can take place via catalytic reactions, i.e. a substance can also be enzymatically bound to the (poly)peptide using an enzyme specifically modifying only said (poly)peptide. Examples of such enzymes are specific kinases, phosphatases or ligases An example of an enzyme known to be specifically associated with SGs is the peptidylglycine alpha-amidating monooxygenase (PAM), which acts both as a monooxygenase and a lyase and which is necessary for amidating the carboxyterminus of many neuropeptide hormones, such as vasopressin, oxitocin, substance P or gastrin. International Patent Application WO 2004/104588 discloses a method for covalently labeling acyl carrier protein (ACP) fusion proteins with a wide variety of different labels. The method relies on the transfer of a label from a coenzyme A type substrate to an ACP fusion protein using a holo-acyl carrier protein synthase (ACPS) or a homologue thereof. The method allows detecting the fusion protein, both in vitro and in vivo, by attaching molecules to the fusion proteins that introduce a new physical or chemical property to the fusion protein.

The (poly)peptide to which at least three different substances specifically and covalently bind is preferably an enzyme which undergoes an inactivation process when acting on a substrate (suicide inactivation) caused by the substrate or a cleavage product thereof covalently binding to the enzyme. Until now, several (poly)peptides exerting the above properties are known. Prominent examples are the SNAP-tag (described in International Patent Applications WO 02/083937 and WO2006/021553) and the HaloTag, both also described in detail further below. The SNAP-tag for example comprises an enzyme forming a covalent bond with an alkyl group in its substrate, a guanine base derivative. A number of different substrates can be generated for the SNAP-tag by linking a desired compound, e.g. a fluorophore, to the guanine base derivative. Further examples of naturally occurring enzymes undergoing a suicide inactivation are xanthine oxidoreductase, quinoprotein alcohol dehydrogenases and DNA methyltransferases.
In one further alternative, the (poly)peptide to which at least three different substances specifically and covalently bind comprises a binding site reactive to certain chemical compounds. A prominent example is the In-Cell tetracysteine motif Cys-Cys-Xaa-Xaa-Cys-Cys where Cys equals cysteine and Xaa equals any amino acid other than cysteine (further described below) comprised in (poly)peptides. This motif is rarely seen in naturally occurring proteins allowing specific fluorescent labelling of recombinant proteins fused to the TC-Tag by covalent binding of fluorescently labelled biarsenic compounds.

The term "fusion protein" refers to chimeric proteins consisting of sequences derived from at least two different molecules. According to the present invention, in exemplary fusion proteins, a (poly)peptide specific for SGs is fused to a (poly)peptide having a binding site common for at least three different substances A, B and C covalently binding to said common binding site. Fusion may be performed by any technique known to the skilled person, as long as it results in the in frame fusion of the nucleic acid molecules encoding the components of the fusion proteins of the invention. Fusion of the components may be effected in any order. Conventionally, the generation of a fusion protein from two or more separate (poly)peptides or domains is based on the "two-sided splicing by overlap extension" described in Horton R., et al., 1989, Gene 77:61-68. The fragments coding for the (poly)peptides are generated in two separate primary PCR reactions. The inner primers for the primary PCR reactions contain a significant, approximately 20 bp, complementary region that allows the fusion of the two domain fragments in the second PCR. Alternatively, the coding regions may be fused by making use of restriction sites, which may either be naturally occurring or be introduced by recombinant DNA technology.

Fusing a (poly)peptide having a binding site common for at least three different substances with a (poly)peptide specific for SGs is done in order not to affect or impair the biological function of any one of the component (poly)peptides, especially the one specific for SGs. Otherwise, the altered biological function may lead to a different behaviour of SGs and thus to no reliable results upon practising the method of the present invention. The skilled person is aware of methods for testing the biological activity of a fusion protein and comparing it with that of each separate component.

The term "capable of penetrating the cell membrane" denotes the ability of substances to translocate through the cell membrane in order to be able to bind to its target structure within the cell. Several substances are capable of penetrating the cell membrane per se, e.g. lipophilic substances or (poly)peptides such as the HIV-1 TAT protein, the herpes simplex virus 1 (HSV-1) DNA-binding protein VP22, and the Drosophila Antennapedia (Antp) homeotic transcription factor, whose amino acid sequences and structures have characteristics that allow their uptake into the cell.

Substances can be "detectable" by different means. Exemplary substances emit any kind of detectable radiation such as light (e.g. fluorescent or chemiluminescent light). "Detectable by different means" denotes different principles of detection, e.g. visible light vs. UV-light or, e.g. in the case of different fluorescent substances, basically similar but still distinguishable detection properties.

The term "saturating" refers to the provision of a sufficient amount of a substance so that all binding sites on the (poly)peptide having a binding site common for at least three substances A, B and C are occupied. In other words, with an excess of any one of substances A, B or C, binding to said (poly)peptide is quantitative.

The term "allowing that unbound substance B is removed from the cell or removing unbound substance B from the cell" denotes a process wherein the cell is incubated and the substance is either actively removed by the cell through transport processes or the substance is diffusing out of the cell due to concentration differences. Alternatively, the experimenter can actively perform the process by changing the culture medium once or more often, or by washing the cell.

The term "becoming available for binding after step (b)" denotes the process of expressing and forming new SGs after substance B has bound to so far expressed SGs. New SGs may thus form during or after unbound substance B is removed.

The present inventors have surprisingly found that SGs can be reliably differentially labeled according to their age by applying a technique based on the detectability of substances having a common binding site in or on SGs. The method of the present invention relies on the fact that several substances may bind to the same or at least overlapping binding sites on a (poly)peptide. This property of (poly)peptides, be it (poly)peptides such as the SNAP-tag forming part of a fusion protein further comprising a (poly)peptide specific for SGs, was used for the first time in connection with the differential labeling of SGs. By contacting SGs with substances specifically and covalently binding to fusion proteins comprising (poly)peptides specific for SGs, essentially the entire population of SGs having formed until the application of the labeling substance can be labeled. Afterwards potentially remaining binding sites are saturated and unbound substance is removed either passively (by diffusion and/or dilution) or actively from the cell. During and after the time allocated for the substance to be removed, the cell can express and form new SGs comprising a fusion protein comprising a (poly)peptide specific for SGs and a (poly)peptide having a binding site common for at least three substances A, B and C covalently binding thereto. Since these SGs have not yet been contacted with a detectable substance they have not yet been labeled. In a second labeling step, the newly formed SGs are labeled according to the first population labeled but with a substance detectable by different means. For example, if substance A comprises a fluorescent moiety detectable upon excitation with a certain wavelength, substance C could comprise either a fluorescent moiety detectable upon excitation with a different wavelength than substance A or one emitting fluorescence at a different wavelength than substance A upon excitation with the same or a different wavelength.

Duncan et al. (2003) have analysed the age-dependent distribution of SGs within chromaffin cells. This was effected by conjugating the SG-specfic protein ANF to the timer protein dsRed-E5 (the "GFP-timer) which progressively shifts its fluorescence emission from green to yellow and finally to red within 16 hours on average in context with the protein's maturation process. During maturation, a GFP-line fluorophore is modified to result in a red fluorophore (Terskikh et al., 2000). The spatiotemporal separation of newly synthesized granules was apparent from the vesicles less than 3 days old being yellow; older vesicles appeared red and younger ones green. One major drawback of said method is that the GFP-timer molecules do not age in the same time range or to the same extent. Thus, whereas some GFP-timer molecules change their emission wavelength with a certain speed, some other equally otd GFP-timer molecules, change their emission wavelength with a different velocity or even not at all. As a consequence, GFP-timer molecules present in SGs of the same age do not necessarily exert the same emission wavelength, thus limiting the accuracy with which the age of SGs can be assessed. Furthermore, on average, the GFP-timer changes its emission wavelength over a certain time. Accordingly, in order to detect ageing of the SGs, the emission wavelengths have to have a certain distance in the spectrum in order to be distinguished. This results in the impossibility to examine the behavior of SGs the ages of which differ by less than the time necessary for the GFP-timer to shift its emission wavelength to one distinguishable from the initial wavelength. On the other hand, if a time frame longer than the maximum time necessary for a shift from green to red is envisaged for experiments, all GFP-timer molecules in any SGs will emit red light so that no distinction of differently aged SG populations is possible any more. Accordingly, the time frame at disposal for experiments or screens is comparably narrow whereas the method of the present invention does not only provide for controlled experiments but also for a flexible time frame. The experimenter can decide how many populations of SGs to label and in which time period. Furthermore, as long as a sufficient number of substances binding to said common binding site and detectable by different means is available, more than two differently aged SG populations can be labeled.

The present invention was put into practice on the example of insulin fused to the SNAP-tag (see the appended Examples). Fluorescent labeling was carried out by applying fluorescent substrates of the SNAP-tag to the cell which were covalently bound. The prior art had difficulties with labeling secretory granules with tagged insulin. As stated by Tsuboi et al. 2006, the targeting of insulin-GFP chimeras to secretory granules has proved problematic.
Furthermore, as regards the system of enzymatic covalent binding used in the present invention, the SNAP technology used as an example relies on the reactivity of a reduced cysteine in the SNAP polypeptide with the substrate. Cysteines of secretory proteins, however, are typically oxidized. Thus, the skilled person would have expected that proper folding of hINS-SNAP may have been impaired by the single reactive cysteine in the SNAP tag and result in one of following possibilities: 1) unfolded hINS-SNAP could have been degraded in the endoplasmic reticulum; 2) improperly folded hINS-SNAP could have been mis-targeted to organelles other than the SGs; 3) the reactive cysteine in hINS-SNAP could have driven the formation of disulphide bridges with free cysteines in other molecules. The formation of such dimers, in addition to promoting the degradation and mis-targeting of hINS-SNAP, could have also precluded its reactivity with the substrate. In summary, the skilled person would not have been motivated to try to label insulin with a fluorescent substance let alone use the properties of covalently modified enzymes for labelling with a reasonable expectation of success.

It is of note that, in context with the present invention, (poly)peptides specific for certain types of SGs do not have to be chosen to match with the type of SG expressed in the cell of interest examined. Upon transfection with the fusion protein of the invention comprising a (poly)peptide specific for SGs, said fusion protein will be expressed and directed to SGs even if the particular cell does normally not produce said (poly)peptide in its SGs specific for different substances. For example, a nucleic acid molecule encoding a fusion protein comprising insulin can be transfected into a chromaffin cell with the expectation that the fusion protein will be expressed and directed to the SGs normally containing epinephrine or norepinephrine.

The method of the present invention enables for studying the behaviour of SGs in the cell during cellular processes as well as upon stimulation with radiation or substances such as hormones, cytokines, low molecular weight substances such as sugars, ions etc.

In a second aspect, the present invention relates to an in vitro method of investigating in a cell the effect of a stimuls on secretory granules (SGs) differentially labelled according to their age as characterised in the appended claims comprising (a) contacting a cell capable of forming SGs and expressing a fusion protein comprising (i) a (poly)peptide specific for SGs and (ii) a (poly)peptide having a binding site common for at least three different substances A, B and C covalently binding thereof, wherein said substances are capable of penetrating the cell membrane and wherein at least substances A and C are detectable by different means with a. a substance A targeting said binding site; and saturating the remaining binding sites by contacting the cell with a substance B which blocks the binding sites which were not bound by substance A or b. a substance B which blocks said binding site in an amount sufficient to saturate said binding sites (b) allowing that unbound substance B is removed from the cell or removing unbound substance B from the cell (c1) applying a stimulus to the cell, (d1) contacting the cell with a substance C targeting said binding site on the fusion protein expressed during and/or after step (c), and (e1) detecting for the presence of substance A and/or C and/or monitoring the number, size, motility, location in the cell, and/or secretion of the differentially labelled SGs obtained in step (a) and (d1), or (c2) contacting the cell with a substance C targeting the binding site on the fusion protein expressed during and/or after step (c), (d2) applying a stimulus to the cell; and (e2) detecting for the presence of substance C and/or monitoring the number, size, motility, location in the cell and/or secretion of the differentially labelled SGs obtained in step (a) and (c2).

The term "a stimulus" describes the result of contacting or treatment of a cell with radiation, such as light, heat, radioactive radiation, or substances such as hormones, cytokines, low molecular weight substances such as sugars, ions or proteins.

The term "monitoring the number, size, motility, location in the cell and/or secretion" refers to tracking the behavior of SGs to monitor the development of the number, size, location, motility and/or secretion of SGs. It is envisaged that certain stimuli are capable of inducing or inhibiting the expression, growth, motility and/or secretion of SGs, which would be detectable in an alteration in any one of the above properties. The detection of said properties is facilitated by the detectable moiety comprised in the substances used for labeling.

In a preferred embodiment of the second aspect, the stimulus is a test agent and the effect to be investigated is the ability of the test agent to induce the formation of secretory granules (SGs) or their secretion or both in a cell, wherein the method further comprises step (f) comparing the results obtained in step (e), i. e. (e1) or (e2), with those obtained for a reference cell not having been contacted with the test agent but otherwise treated equally, wherein an amount of substance A and/or C detected in the cell which is different than that in the reference cell is indicative of the ability of the test agent to affect the formation and/or the secretion of SGs in the cell.

A "test agent" can be any chemical including a substance as defined under "stimulus".

In a preferred embodiment of the invention, the SG is an insulin granule and the (poly)peptide in step (a)1. is specific for insulin granules.

β-cells of pancreatic islets are the endocrine cells that produce insulin, the most important hormone for the control of glucose homeostasis in vertebrates. Insulin is stored in and secreted from the SGs of pancreatic β-cells. Glucose stimulates both the Ca²⁺-dependent exocytosis of insulin SGs as well as the biosynthesis of insulin and other SG components (Guest et al., 1989; Guest et al., 1991), including chromogranin A, and the prohormone convertases 1/3 (PC1/3) (Alarcon et al., 1993; Martin et al., 1994) and 2 (PC2) (Martin et al., 1994). Each β-cell stores ~10⁴ insulin secretory granules (Bratanova-Tochkova et al., 2002; Rorsman and Renstrom, 2003). Less than 5% of them are readily releasable, i.e. undergo exocytosis during the first phase insulin secretion, which lasts up to 10 minutes after the elevation of blood glucose >5 mM. The remaining >95% of the granules belong to the reserve pool.

Other secretory granules are for instance those of α-cells of the pancreatic islets that secrete the peptide hormone glucagon or those of the pituitary cells that secrete growth hormone or prolactin.

In accordance with the invention, the (poly)peptide of (ii) is a SNAP-tag™, a HaloTag® or an In-Cell Tetracysteine Tag.

A SNAP-tag™ (Covalys) can be used for covalent and specific linkage of almost any chemical substance to a protein of interest both in vitro and within living cells (Keppler et al., 2003). The SNAP-tag technology is based on the human form of the DNA repair protein O6-alkylguanine-DNA alkyltransferase (AGT). The physiological function of AGT is the removal of alkyl groups from guanine bases of DNA to avoid mutations during cell division. AGT undergoes a classical suicide reaction as the cysteine residue in its active site forms a covalent and stable thioether bond with the alkyl group, allowing the release of the de-alkylated, original guanine base (Keppler et al., 2003; Daniels et al., 2000). It was found that AGT can accept any chemical compound wlien it is linked to the guanine base through a benzyl group. By direct evolution experiments and protein engineering with hAGT a smaller enzyme was designed having much faster reaction kinetics and devoid of interaction with other biomolecules such as double-stranded DNA (Juilierat et al., 2003). The reaction mechanisms of the SNAP-tag system allow the covalent and specific attachment of target proteins to any desired compound or solid surface and is useful, inter alia, for in vitro and in vivo labelling in the method of the present invention.

The HaloTag® Interchangeable Technology (Promega) enables for the rapid, site-specific and irreversible labelling of proteins in living cells or in vitro. The technology is composed of HaloTag protein, a genetically modified dehydrolase, which is fused to a protein of interest, and synthetic HaloTag ligands. Fusion proteins can be expressed at the C- or the N-terminus of the HaloTag®-Protein. The formation of a covalent bond between the HaloTag protein and synthetic ligands can impart a variety of functionalities, including fluorescence, affinity tags and direct attachment to a solid surface.

The labelling reagents available for labelling using the In-Cell tetracysteine motif bind a tetracysteine motif consisting of Cys-Cys-Xaa-Xaa-Cys-Cys where Cys equals cysteine and Xaa equals any amino acid other than cysteine. This motif is rarely seen in naturally occurring proteins allowing specific fluorescent labelling of recombinant proteins fused to the TC-Tag. In the TC-FLAsH™ TC-ReAsH™ II In-Cell Tetracysteine Tag Detection Kits (all Invitrogen), the optimized Cys-Cys-Pro-Gly-Cys-Cys tetracysteine motif is used as this motif has been shown to have a higher affinity for and more rapid binding to biarsenic compounds as well as enhanced stability compared to other characterized motifs.

In a more preferred embodiment of the invention, substance B binding to the SNAP-tag is BTP.

In a further preferred embodiment of the invention, substance A binding to the SNAP-tag is TMR-Star, BG-505, BG-430 or BG-DAF.

In another preferred embodiment of this embodiment, substance C binding to the SNAP-tag is TMR-Star, BG-505, BG-430 or BG-DAF. The substance applied in an experiment as substance C is different from the substance applied as substance A.

The substrates available for the SNAP-tag are listed in table 1 below. Further substrates are listed in the international patent application WO2006/114409.

**Table 1: substrates for the SNAP-tag and their properties**

| Substance | Excitation/emission maximum | fluorophore | remarks |
|---|---|---|---|
| SNAP-cell 360 | 357 nm/437 nm | aminomethylcoumarin | based on cell-permeable CP-360, suitable for use with DAPI filter sets |
| SNAP-cell 430 | 421nm/444nm and 484nm | intense blue photostable fluorophore | based on cell-permeable BG-430 |
| SNAP-cell 505 | 504nm/532nm | photostable green fluorophore in the fluorescein filter window | based on cell-permeable BG-505 |
| SNAP-cell DAF | 500nm/532nm | fluorescein | based on cell-permeable BG-diacetyl fluorescein (DAF). DAF is converted to fluorescein once it has entered the cell |
| NAP-cell TMR-Star | 554nm/ 580nm | photostable tetramethylrhodamine | based on cell-permeable TMR-Star |
| SNAP-block Cell | based on bromothenylpteridine (BTP). BTP is highly cell permeable and reacts irreversibly with the SNAP-tag, blocking it and preventing it from reacting further with SNAP-cell substrates. It is useful to generate inactive controls in cell labeling experiments and also as a blocker of nascent SNAP-tag fusion proteins in pulse chase applications. | | |
| SNAP-biotin | enables the biotinylation of SNAP-tag fusion proteins in solution or in live cells. Applications include capture of fusion proteins on streptavidin-coated surfaces for interaction assays, detection of proteins in fixed cells using streptavidin fluorophore conjugates. | | |

In a further preferred embodiment of the second aspect, the stimulus is a chemical substance, e.g. glucose, acetylcholine, glucagon-like peptide 1, exenatide, sulfonylureas, endosulfine, meglitinide, potassium, catecholamines, glucagon, somatostatin, ghrelin, diazoxide.

In another preferred embodiment of the invention, the detection or monitoring is effected by detecting fluorescence emission after excitation at a proper wavelength, chemiluminescence or light absorbance.

In connection with this embodiment of the present invention, fluorescence emission is detected using a fluorescence microscope.
A fluorescence microscope is a light microscope used to study properties of organic or inorganic substances using the phenomena of fluorescence and phosphorescence instead of, or in addition to, reflection and absorption. The specimen is illuminated with light of a specific wavelength (or wavelengths) which is absorbed by the fluorophores, causing them to emit longer wavelengths of light (of a different color than the absorbed light). The illumination light is separated from the much weaker emitted fluorescence through the use of an emission filter. Typical components of a fluorescence microscope are the light source (Xenon or Mercury arc-discharge lamp), the excitation filter, the dichroic mirror (or dichromatic beamsplitter), and the emission filter (see figure below). The filters and the dichroic mirror are chosen to match the spectral excitation and emission characteristics of the fluorophore used to label the specimen. Most fluorescence microscopes in use are epi-fluorescence microscopes (i.e.: excitation and observation of the fluorescence are from above (epi) the specimen). These microscopes have become an important part in the field of biology, opening the doors for more advanced microscope designs, such as the confocal laser scanning microscope and the total internal reflection fluorescence microscope (TIRF).

In a more preferred embodiment of this embodiment, the detection for the presence of substance A and/or C is quantitative.
Quantitative detection can be effected with specialized software, e.g. ImageJ, Metamorph, Motiontracking, IPLab and others.

In a further preferred embodiment of the invention, the fusion protein is expressed after introducing a nucleic acid encoding said fusion protein into the cell. Introduction of nucleic acid molecules into cells can be effected by transfecting the nucleic acids. Commonly used methods for transfection comprise but are not restricted to transfection using lipofection, calcium chloride, polyethylenimine derivatives, DEAE-dextran, gene guns, electroporation, nucleoporation, magnetofection, microinjection, and viral vectors.

In another preferred embodiment of the first aspect of the invention, the method further comprises washing the cell after step (a), (b), (c) and/or (d). In a further preferred embodiment of the second aspect, the method further comprises washing the cell after step (a), (b), (c1), (d1), (c2) and/or (d2).

Washing can be performed either with culture medium or with PBS or any buffer suitable to wash cells such as Ringer solution and HEPES buffer. The duration of washings can vary from fast rinse to several minutes (e. g. 2 to 10 minutes). Each single of the above method steps or any combination of method steps can be followed by a washing step. After each step, one or more washing steps can be effected.

In a more preferred embodiment of the invention, the (poly)pepfide specific for SGs is insulin, phogrin, ICA512, carboxipeptidase E/H, chromogranin A, chromogranin B, secretogranin II, protein convertase 1 or 2, amylin or another SG-specific neuropeptide endocrine hormone such as growth hormone, prolactin, ANP, and NPY.

In a further preferred embodiment of the invention, the different means of detection of substances A and C are different excitation and/or emission wavelengths.

In another preferred embodiment of the invention, substance A or C or both comprise 1. a moiety specifically binding the binding site on the fusion protein and 2. a detectable moiety. Both properties, i.e. a specific binding site on the fusion protein and the property conferring detectability, can be present in one moiety.

In a different preferred embodiment of the first aspect, the method further comprises contacting the cell with a substance D targeting said binding site on the fusion protein becoming available for binding after step (d) and after allowing that unbound substance C is removed from the cell or after removing unbound substance C from the cell.

With this embodiment of the present invention, it is possible to label three different populations of SGs of different ages.
As described above, upon availability of a sufficient number of substances detectable by different means and binding to a common binding site on said fusion protein, more than three, e.g. four, five or six, different populations of SGs can be labelled according to their age by repeating steps (c) and (d) and optionally (b) with the respective substances. Accordingly, for four different substances, the step according to step (c) comprises allowing that unbound substance D (or optionally B if step (b) is applied prior to the equivalent of step (c)) is removed from the cell or removing unbound substance D (or optionally B if step (b) is applied prior to the equivalent of step (c)) from the cell; and the step according to step (d) comprises contacting the cell with a substance E targeting said binding site on the fusion protein becoming available for binding after the preceding step.

In a different embodiment, the present invention relates to a method for detecting a substance secreted from uniformly or differentially labelled SGs according to their age as characterised in the appended claims comprising: (a) separating a cell capable of forming SGs and expressing a fusion protein comprising (i) a (poly)peptide specific for SGs and (ii) a (poly)peptide having a binding site common for at least three different substances A, B and C covalently binding thereto, wherein said substances are capable of penetrating the cell membrane and wherein at least substances A and C are detectable by different means and (I) treated according to the method of any one of claims 1 to 14; from its culture medium; (b) detecting in the medium the presence of any detectable substance applied for labeling the SGs, if any; wherein the detection of one or more substances in the medium indicates that secretion from SGs has taken place.

In contrast to the methods described further above, where differentially labeled SGs are detected in the cell, the present method investigates which SGs are secreted into the medium. The present method is thus able to detect the secretion of SGs of different ages as well as secretion of SGs caused by a stimulus.

The present invention furthermore refers to a non-human transgenic host as characterised in the appended claims comprising a polynucleotide encoding a fusion protein comprising (i) a (poly)peptide specific for SGs and (ii) a (poly)peptide having a binding site common for at least three different substances A, B and C covalently binding thereto.

In a preferred embodiment, the non-human transgenic host is an animal.
The transgenic non-human animal may, for example, be a transgenic zebrafish, mouse, rat, hamster, dog, monkey, rabbit, pig, or cow. Preferably, said transgenic non-human animal is a mouse.

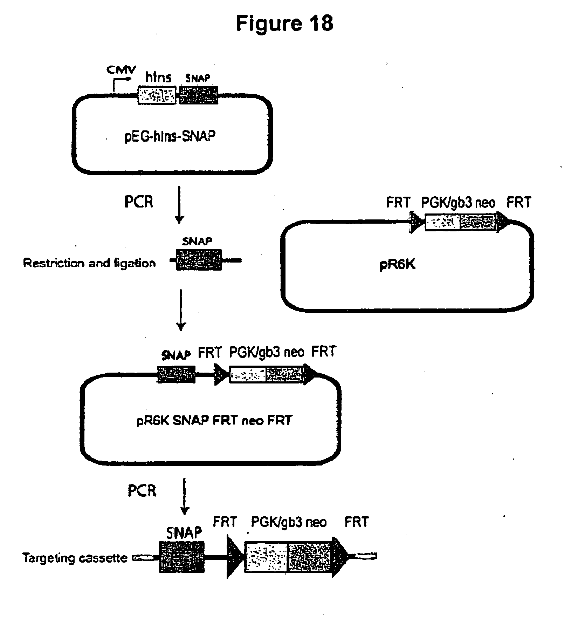

A method for the production of a transgenic non-human animal, for example transgenic mouse, comprises introduction of a polynucleotide encoding the fusion protein of the invention or targeting vector into a germ cell, an embryonic cell, stem cell or an egg or a cell derived therefrom (see Figure 18 for an exemplary strategy). The non-human animal, in particular primary beta-cells or islets isolated from said animal, optionally placed in an environment different from their natural one (e. g. the pancreas for islets), can be used in the methods of the invention described above. For example can a physiological environment for islet development be created by transplantation of isolated islets into the eyes of mice (Speier et al., 2008).
Production of transgenic embryos and screening of those can be performed, e.g., as described by A. L. Joyner Ed., Gene Targeting, A Practical Approach (1993), Oxford University Press. The DNA of the embryonic membranes of embryos can be analyzed using, e.g., Southern blots with an appropriate probe; see supra. A general method for making transgenic non-human animals is described in the art, see for example WO 94/24274. For making transgenic non-human organisms (which include homologously targeted non-human animals), embryonal stem cells (ES cells) are preferred. Murine ES cells, such as AB-1 line grown on mitotically inactive SNL76/7 cell feeder layers (McMahon and Bradley, Cell 62:1073-1085 (1990)) essentially as described (Robertson, E. J. (1987) in Teratocarcinomas and Embryonic Stem Cells: A Practical Approach. E. J. Robertson, ed. (Oxford: IRL Press), p. 71-112) may be used for homologous gene targeting. Other suitable ES lines include, but are not limited to, the E14 line (Hooper et al., Nature 326:292-295 (1987)), the D3 line (Doetschman et al., J. Embryol. Exp. Morph. 87:27-45 (1985)), the CCE line (Robertson et al., Nature 323:445-448 (1986)), the AK-7 line (Zhuang et al., Cell 77:875-884 (1994)). The success of generating a mouse line from ES cells bearing a specific targeted mutation depends on the pluripotence of the ES cells (i. e., their ability, once injected into a host developing embryo, such as a blastocyst or morula, to participate in embryogenesis and contribute to the germ cells of the resulting animal). The blastocysts containing the injected ES cells are allowed to develop in the uteri of pseudo-pregnant non-human females and are born, e.g. as chimeric mice. The resultant transgenic mice are chimeric for cells having either the recombinase or reporter loci and are backcrossed and screened for the presence of the correctly targeted transgene(s) by PCR or Southern blot analysis on tail biopsy DNA of offspring so as to identify transgenic mice heterozygous for either the recombinase or reporter locus/loci.

The transgenic host can also be a cell, i.e. a prokaryotic or eukaryotic cell. In connection with the present invention, a transgenic cell is a cell stably transfected (eukaryotic cells) or transformed (prokaryotic cells) with a nucleic acid encoding the fusion protein of the invention. Transfection techniques, such as lipofection or transfection using CaCl₂, and transformation techniques, e.g. using electroporation or heat shock, are well-known to the skilled person.

In a preferred embodiment, the SG is an insulin granule and the (poly)peptide of item (i) is specific for insulin granules.

In accordance with the present invention, the (poly)peptide of item (ii) is a SNAP-tag, a HaloTag or an In-Cell Tetracysteine Tag.

In a more preferred embodiment of the invention, substance B binding to the SNAP-tag is BTP.

In a further preferred embodiment of the invention, substance A binding to the SNAP-tag is TMR-Star, BG-505, BG-430 or BG-DAF.

In another preferred embodiment, substance C binding to the SNAP-tag is TMR-Star, BG-505, BG-430 or BG-DAF. The substance applied in an experiment as substance C is different from the substance applied as substance A.

In a preferred embodiment of the invention, the (poly)peptide specific for SGs is insulin, phogrin, ICA512, carboxipeptidase E/H, chromogranin A, chromogranin B, secretogranin II, protein convertase 1 or 2, amylin or another SG-specific neuropeptide endocrine hormone such as growth hormone, prolactin, ANP, and NPY.

In another preferred embodiment, the different means of detection of substances A and C are different excitation and/or emission wavelengths.

In another preferred embodiment of the invention, substance A or C or both comprise 1. a moiety specifically binding the binding site on the fusion protein and 2. a detectable moiety.

In a preferred embodiment of the methods of the present invention, the cell capable of forming SGs is a primary β-cell from islets isolated from the transgenic animal, preferably the transgenic mouse of the invention.

In another preferred embodiment of the methods according to the first and second embodiment of the present invention, the method further comprises contacting the cell to be subjected to step (a) with a substance B which blocks the binding sites on said (poly)peptide already existing (expressed) in the cell and allowing that unbound substance B is removed from the cell or removing unbound substance B from the cell.
The present inventors found that a first step of blocking the binding sites on the labelled fusion protein according to the invention enables for a more accurate age determination of the SGs labelled subsequently. Thus, not only a distinction of the ages of differentially labelled SGs is possible but also an exact determination of the age of the SGs labelled first.
After contacting the cell with substance B, surplus substance B needs to be removed in order to avoid that binding sites in SGs formed after application of substance B are also blocked and thus not available any more for binding to substance A. Newly forming SGs can be labelled and the time frame chosen for letting new SGs be formed determines a substantially uniform age of the SGs subsequently labelled with substance A.

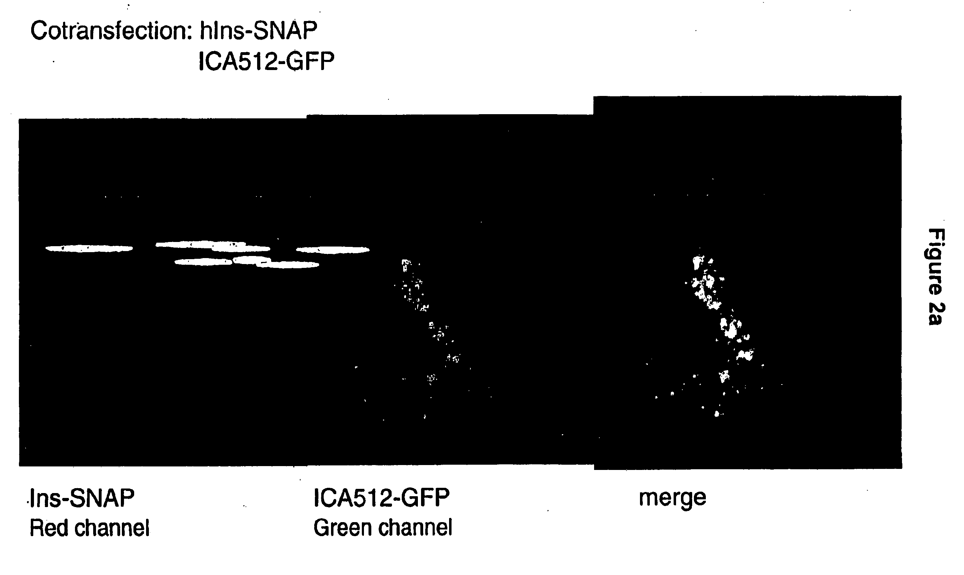

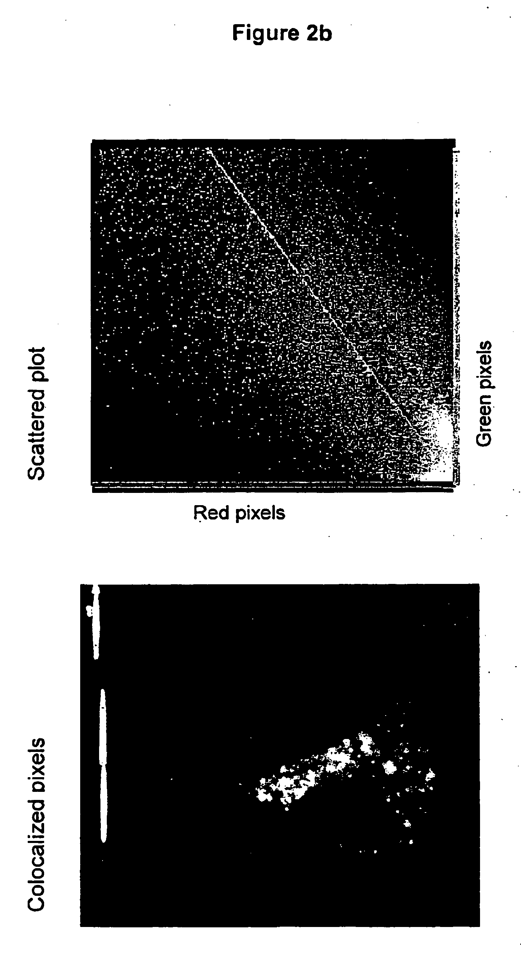

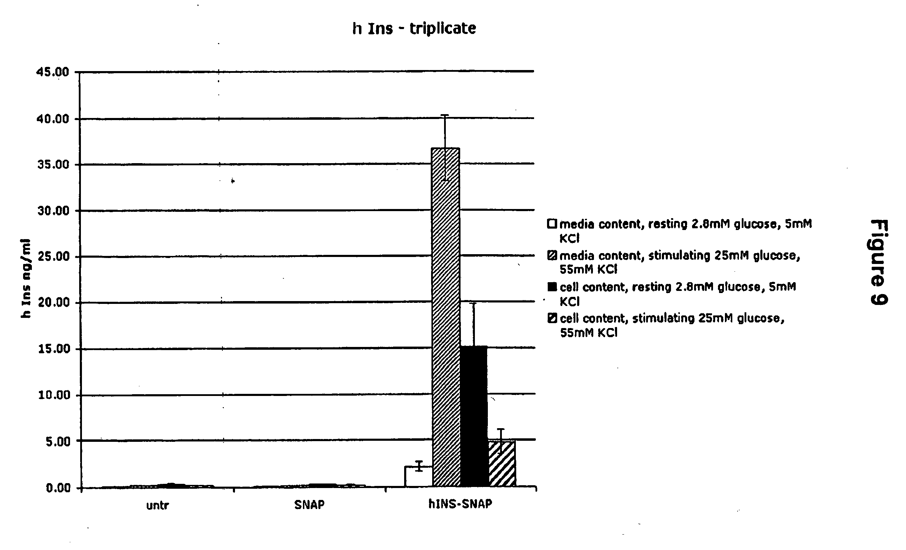

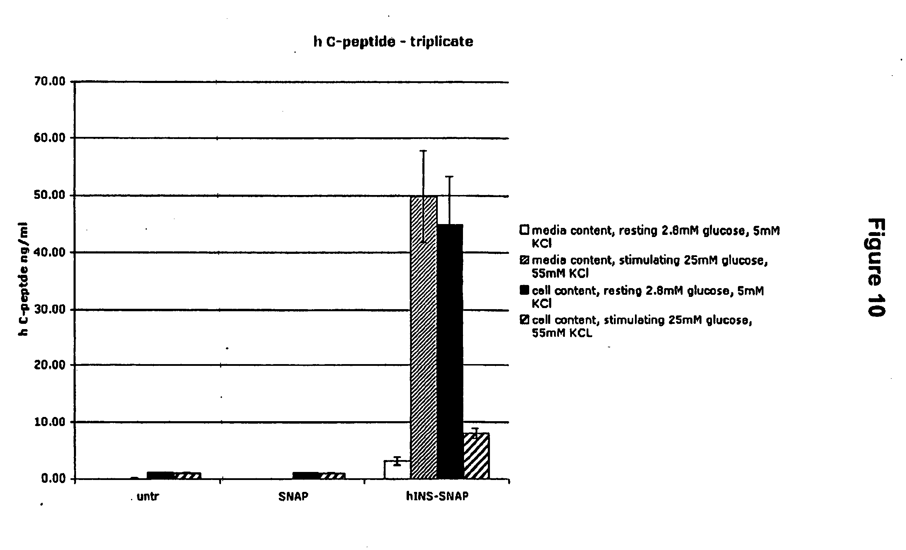

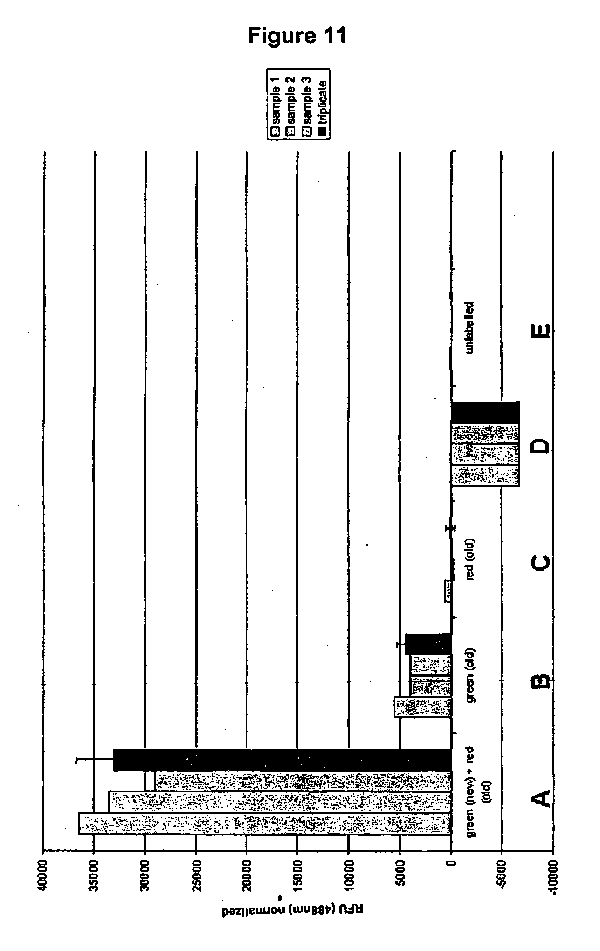

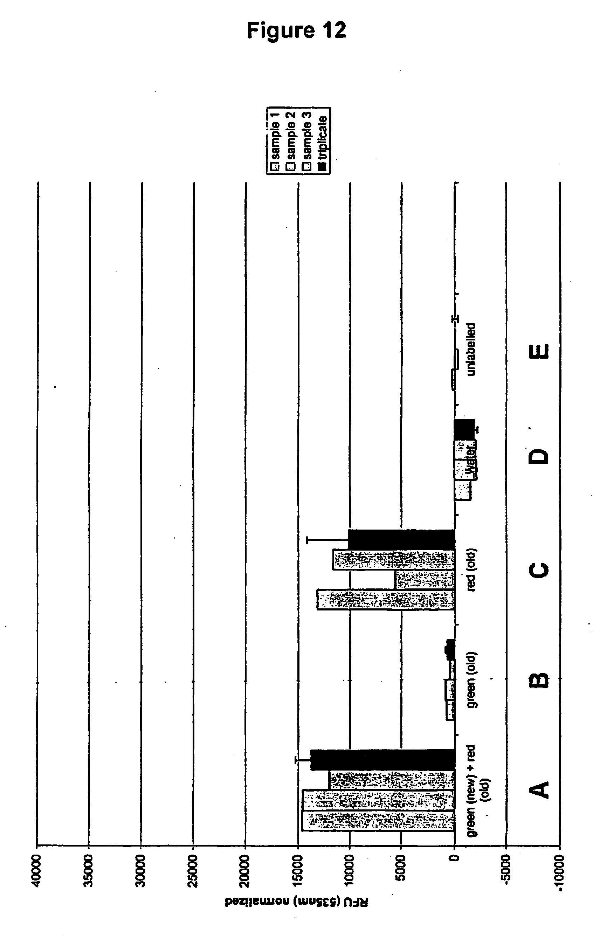

The figures show:

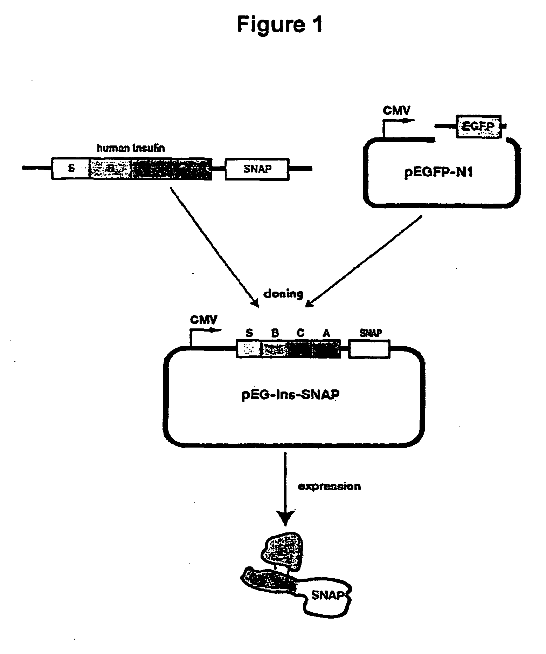

**Figure 1****: Flow chart of the cloning strategy.**
   cDNA of the human insulin (NCBI Genbank accession number: NM 000207), including the signal peptide sequence (S) and the coding sequences for B and A chains together with C peptide were cloned in frame with the SNAP tag. The resulting construct was cloned in pEGFP-N1 vector (NCBI GenBank accession number: U55762), generating the expression vector pEG-Ins-SNAP. S - signal peptide sequence, B - B chain sequence, C - C peptide sequence, A - A chain sequence, CMV - cytomegalovirus promoter, SNAP - SNAP tag, EGFP - enhanced GFP.
**Figure 2****:**
   (a) Co-localization of hINS-SNAP and ICA-512-GFP in insulinoma INS-1 cells.
      INS-1 cells were co-transfected with plasmids bearing hIns-SNAP and ICA-512-GFP (Trajkovski et al, 2004). hIns-SNAP was labeled with TMR-Star and the localization of both proteins was observed with confocal microscopy.
   (b) Co-localized pixels (in white, left panel) for both red and green fluorescence channels in the same cell shown in Figure 2a and scattered plot derived from it (right panel). The scattered plot was generated with ImageJ image analysis software program and shows the clustering of the pixels from both channels, consistent with the co-localization of hINS-SNAP with the SG marker ICA-512-GFP.

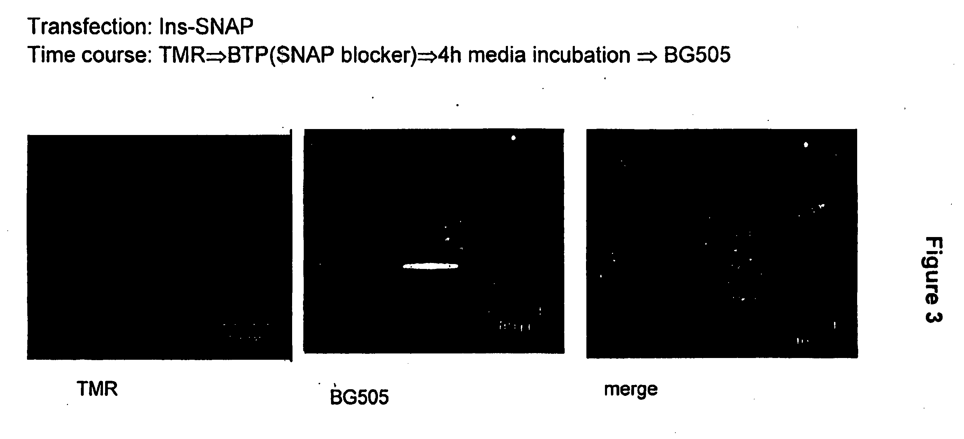

**Figure 3****: In vivo labeling of SGs in hINS-SNAP transfected INS-1 cells.**
   Labelling of living INS-1 cells expressing hINS-SNAP with TMR-Star for 15 minutes was followed by incubation of the cells with BTP for 20 minutes to block remaining binding sites on the SNAP-tag. After additional 4 hours of incubation in medium, cells were then incubated with BG505 for 15 minutes to label the newly generated hINS-SNAP. Old and newly generated SGs could clearly be distinguished based on their differential labelling with TMR-Star (in red) and BG505 (in green), respectively.

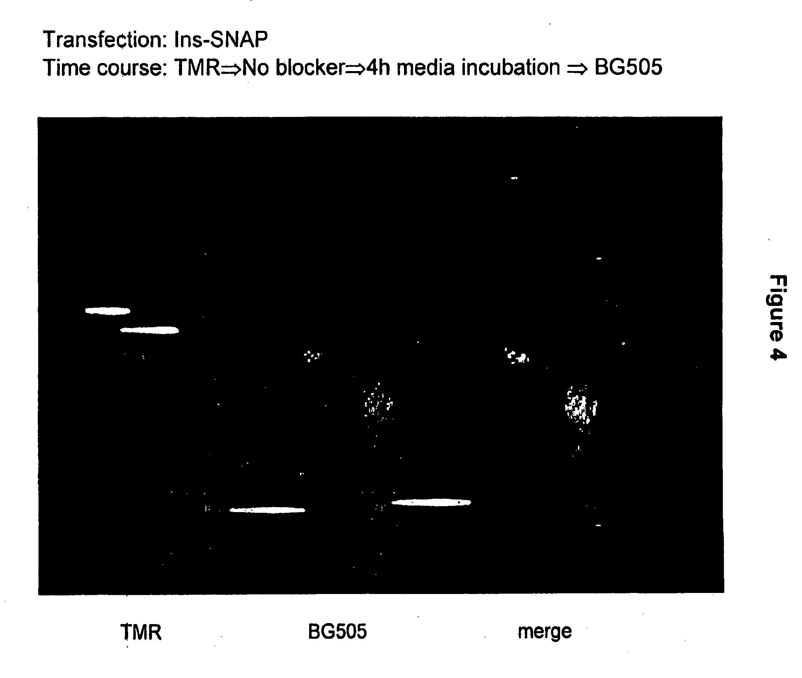

**Figure 4****: In vivo labeling of secretory granules.**
   Labelling of living INS-1 cells expressing hINS-SNAP with TMR-Star for 15 minutes. Following their incubation for additional 4 hours in medium, the cells were then incubated with BG505 for 15 minutes to label the newly generated hINS-SNAP. In this case the omission of the blocking step with BTP prevented the differential labeling of old and newly generated SGs with TMR-Star and BG505, respectively.
**Figure 5****:**
   a) Co-localized pixels of the images shown in Fig. 3 (merge panel) and Fig. 4 (merge panel) using the same procedure described in the legend of Fig. 2b. No co-localization (white pixels) was detected when the blocking step with BTP was interposed between the sequential labelings with TMR-Star and BG505. Conversely, many co-localized pixels were detected when the blocking step with BTP was omitted.
   (b) Scattered plots of the co-localized pixels derived from the images shown in Fig. 3 (merge panel) and Fig. 4 (merge panel). In case of lack of co-localization between the red and the green pixels, two diverging clouds, each parallel to the respective axis, are formed.
**Figure 6****:**
   Measurements of human insulin in the media (white bars) and in the cell extracts (black bars) of resting (R) and stimulated (S) INS-1 cells transfected with hINS-SNAP. The resting buffer contain 0 mM glucose and 5 mM KCI, while the stimulation buffer contained 25 mM glucose and 55 mM KCl. The amounts of human insulin were specifically assessed using the LINCO RIA kit including an antibody that reacts with human, but not with rat insulin. The very low signal detected in the media and cell extracts of wild-type and SNAP transfected INS-1 cells is accounted by the partial reactivity (62%) of this anti-human insulin antibody with bovine insulin, which is found in the fetal bovine serum used for the culturing of INS-1 cells.
**Figure 7****.**
   Insulin stimulation index (SI) of resting (R) and stimulated (S) INS-1 cells transfected with hINS-SNAP. This SI was calculated as follows = (Insulin_{medium}/Insulinₜₒₜₐₗ)_{Stimulation} /(Insulin_{medium}/Insulinₜₒₜₐₗ)_{Resting}. The value corresponding to (Insulin_{medium}/Insulinₜₒₜₐₗ)_{Resting} was equaled to 100%. The increased fraction of hINS-SNAP released from stimulated cells is consistent with its proper targeting to SGs and its regulated secretion.
**Figure 8**
   Fluorescent labelling with TMR-Star (red) in several cells of a INS-1 cell clone (left panel) stably transfected with hINS-SNAP. The fluorescent labelling of wild-type INS-1 cells with TMR-Star is negative (right panel).
**Figures 9** **and** **10**
   Quantification by radioimmunoassay (RIA) of human Insulin (Figure 9) and human C-peptide (Figure 10) in the media and cell extracts of INS-1 cells. INS-1 cells were either not transfected (untr) or transfected with plasmids encoding SNAP or hINS-SNAP. Cells were then incubated with isotonic saline buffer containing either 2.8 mM glucose and 5 mM KCI (resting buffer) or 25 mM glucose and 55 mKCI (stimulating buffer) for 2 hours.
**Figures 11** **and** **12**
   Fluorimetric detection of hINS-SNAP in the media of transfected INS-1 cells, which had been stimulated with the same protocol described in the legend of Figures 9 and 10. hINS-SNAP was labeled with either BG505 or TMR-Star. Figure 11 and Figure 12 show the fluorescence signals detected in the green (emission filter: 535/25 nm) and in the red (emission filter: 590/20 nm) channels, respectively. A) Old and newly generated hlNS-SNAP, i.e. old and newly generated secretory granules (SGs) were labeled with TMR-Star (red hINS-SNAP) and BG505 (green hINS-SNAP), respectively, following the same protocol described in the legend of Figure 3. B) and C). Only old SGs were labeled with either BG505 (green hINS-SNAP; B) or TMR-Star (red hINS-SNAP; C), by omitting the incubation with the second fluorophore after the blocking step with BTP. D) and E). Background fluorescence detected in water (D) or in the media of transfected INS-1 cells, expressing hINS-SNAP, but not labelled with BG505 or TMR-Star(E).
**Figures 13 to 16**
   Double fluorescence labelings for insulin and markers of the ER (protein disulfide isomerase, PDI), early cisternae of the Golgi complex (Golgi matrix protein of 130kDa, GM130), trans-Golgi network (TGN38) and early endosome (early endosome antigen-1, EEA1). The distribution of hINS-SNAP in transfected INS-1 cells was visualized by labeling with TMR-Star, while the distribution of endogenous insulin in not-transfected INS-1 cells and of all other proteins was detected using specific primary antibodies followed by Alexa-conjugated secondary antibodies.
**Figure 17**
   Western blottings on subcellular fractions of hIns-SNAP-transfected INS-1 cells. Post-nuclear supernatants of hIns-SNAP-transfected INS-1 cells were separated on 0.6-2.0 M continuous sucrose density gradients. Aliquots of equal volume of the gradients were collected, the sucrose molarity of each fraction was calculated based on the refraction index, proteins in each fractions were precipitated with organic solvents, and then separated by SDS-PAGE prior to transfer and immunoblotting with antibodies directed against the A-chain of insulin and markers of the insulin SGs carboxypeptidase E (CPE) and islet cell autoantigen 512 (ICA512) and of the early cisternae of the Golgi complex (GM130).
**Figure 18**
   An example strategy for generation of knock-in mlns2-SNAP mouse is depicted. The scheme includes all steps until generation of a targeting cassette for the mouse Ins2 gene.

The examples illustrate the invention:

### Example 1. Cloning strategy

An exemplary method for the differential labeling of insulin secretory granules (SGs) according to their age relies on the expression and correct targeting of a chimeric human insulin (hINS-SNAP) to the SGs of rat insulinoma INS-1 cells, a model system of pancreatic beta cells. This chimeric protein, termed hINS-SNAP, was created by cloning the cDNA encoding preproinsulin in frame with a 20kDa protein - SNAP-tag™.
The SNAP-tag™ is a commercially available (Covalys), highly engineered version of an alkyl guanine DNA alkyl transferase (DNA repair protein). It reacts with benzyl guanine substrates forming a stable, thioether bonds with the substrate. This reaction is highly specific (Tirat at al., Int J Biol Macromol, 2006)*.*
The cloning strategy implied the generation of a hIns-SNAP cassette cloned into the pEGFP-N1 vector using the restriction sites EcoRI and NotI (see Figure 1 for an overview). The portion of pEGFP-N1 encoding EGFP was excised from the vector beforehand. The reason for choosing pEGFP-N1 is that in previous experiments it showed very strong and stable expression in transfected cells.

### Example 2. Co-localization results I

The correct localization of hINS-SNAP to the SGs was assayed in cells transfected with two plasmids, one bearing hINS-SNAP and the other ICA512, the latter being an intrinsic membrane protein of the insulin-containing SGs (Solimena et al., EMBO J., 1996*;* Ort et al., EMBO J., 2001*;* Trajkovski et al., J. Cell Biol., 2004*).* ICA512 was cloned in plasmid bearing EGFP and ICA512-EGFP has been shown to be targeted to the SGs. Figure 2a shows that both hINS-SNAP and ICA512-GFP are targeted to the same organelles, i. e. SGs.
hINS-SNAP was labeled in living INS-1 cells with the cell permeable SNAP fluorescence substrate TMR-Star (red) (Covalys), which has the following characteristics: excitation wavelength 554 nm, emission wavelength 580 nm.
The labeling protocol optimized in our laboratory is as follows. The cells were labeled on the forth day after transfection. The labeling was carried out for 15 min with 2µM TMR (red) in normal media (1ml per well)(RPMI 1640, 1x with L-glutamine (PAA), 10mM HEPES pH 7,4, 10%FBS (Gibco), 1mM Na-Pyruvate (PAA), 2mM L-glutamine (PAA), 50 β2-Mercaptoethanol, 100 U/ml Pen, 100 µg/ml Strep (Penstrep, PAA)), followed by two times washing with PBS. The period after the end of TMR labeling and the fixation was 4 hours. During this time, the cells were incubated in media without SNAP substrates and two additional washes were performed: two times with PBS and two times with media. This period was necessary to allow the unbound TMR-Star to be cleared out from the cells decreasing in this way the background signal during imaging.
Before fixation the cells were washed two times with PBS, then fixed (4%PFA, pH: 7,4 for 10 min at 4°C) and embedded (ProLong Gold antifade reagent with DAPI P36931, Molecutar Probes).
The images were acquired using a Zeiss Axiovert 200M confocal microscope and Zeiss LSM 510 AIM Version 4.0 software (Zeiss Plan-Apochromat 63x/1.4 Oil, Zeiss LSM 510 Scanhead with Photomultiplier Tubes, red channel: LP575, green channel: BP 505-550).

### Example 3. Co-localization results II

The collected images were processed using the image analyzing software (ImageJ, NIH). This program allows the generation of images showing co-localized pixels from the green (emission peak wavelength: 509nm) and red channels (emission peak wavelength: 580 nm) of the acquired images.

Another possibility to present co-localization is the generation of the 'scattered plot' of an image. It this case the pixels of each channel are plotted in relationship to their intensity. When co-localization occurs, the scattered plot, as in this case, has a specific form resembling a cloud around the middle axis (Bolte et al., Journal of Microscopy, Dec 2006) (see Figure 2b).

### Example 4. Labeling of living INS-1 cells

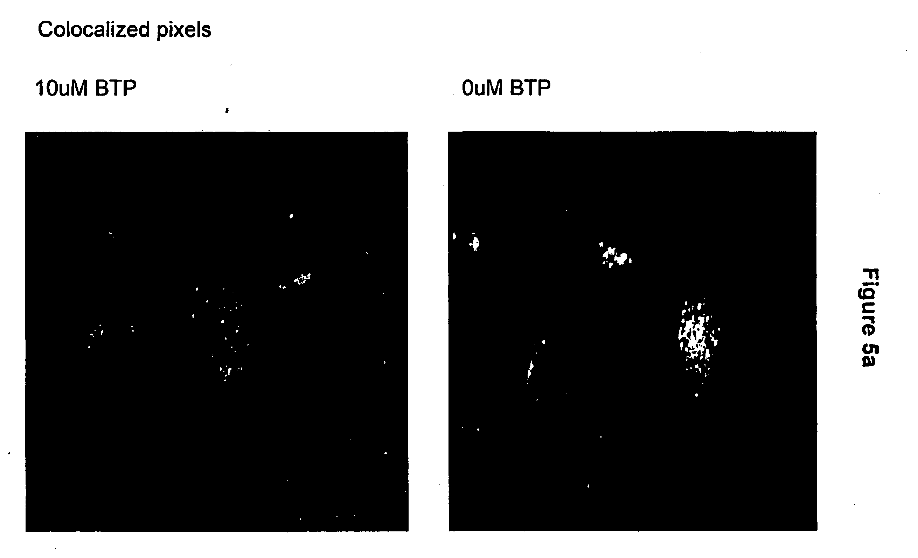

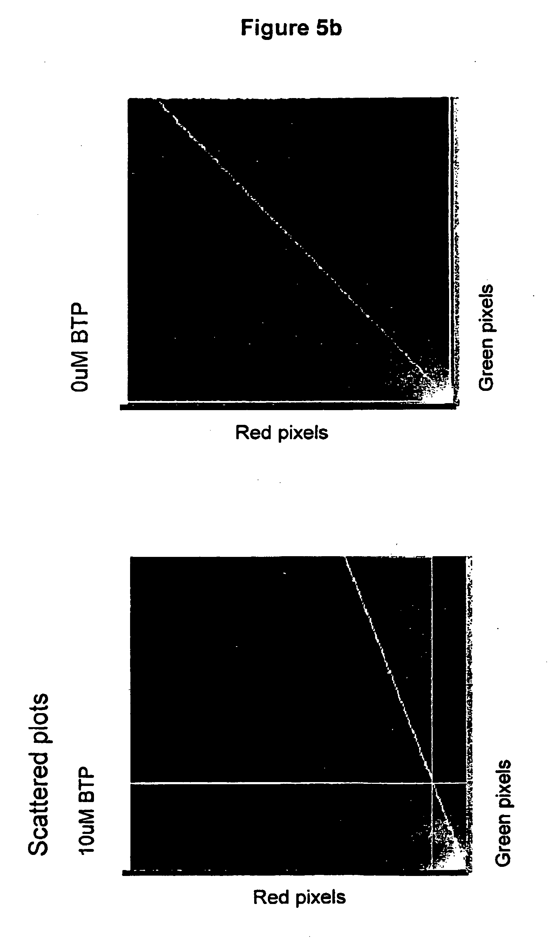

Having determined the correct targeting of hIns-SNAP to insulin secretory granules in fixed INS-1 cells, living INS-1 cells were labeled on the forth day after transfection with phIns-SNAP. The dual color labeling was done according to the following scheme:
The first labeling was carried out for 15 min with 2µM TMR (red) in normal media (1ml per well), followed by 2xPBS wash and 30 min, incubation in media without the SNAP substrate. The labeling with 10 µM BTP lasted 20 min. followed by two washings with PBS and 30 min incubation in media without BTP. The period between the BTP administration and the BG505 (green) administration was 4 hour long, so that any unbound BTP had sufficient time to be cleared out of the cells, thus allowing the labeling of newly synthesized hIns-SNAP. During this period two additional washings were performed: two times with PBS and two times with media.
Cells were then incubated with 10 µM BG505 for 30 min, followed by two washings with PBS, 30 min incubation in media and another PBS washing. After the last washing, the cells were fixed (4%PFA, pH: 7,4 for 10 min at 4°C) and embedded (ProLong Gold antifade reagent with DAPI, P36931, Molecular Probes).
Experiments with different concentrations ranging from 1 to 10 µM of the blocker were compared to control labeling reactions without BTP (see Figures 4 and 5). The results clearly show that the application of the BTP before exposure of cells to the second SNAP-substrate (BG505) allows the detection of two age-distinct populations of granules (red=old granules; and green = new granules; see Figure 3). When the BTP blocker is omitted granules are instead dually tabeled with TMR and BG505, precluding their distinction by age (see Figure 4).

### Example 5. Co-localization results III,IV

Software analysis of the acquired images allowed the generation of images representing the co-localized pixels from both channels and also the characteristic scattered plots. The pixel distribution on the scattered plot obtained from labeling of cells exposed sequentially to TMR, 10 µM BTP, and BG505 has the shape of two independent clouds close to each axis, indicating the absence of co-localization between organelles labeled with TMR and BG505 (see Figure 5b, left panel). In contrast, the scattered plot of images acquired from cells exposed sequentially to the TMR and BG505 fluorophores, without the interposition of the SNAP blocker BTP, retained the shape of a cloud around the middle axis (see Figure 5b, right panel). The calculated Pearson's coefficients (10 µM BTP: 0,515; 0 µM BTP: 0.829) calculated according to Bolte et al., are in agreement with this interpretation.

### Example 6. Spinning disk microscopy

Imaging was performed on living INS-1 cells expressing hIns-SNAP and processed according to the dual-color labeling protocol described in Example 4. After the last PBS wash the cells were incubated in media and observed by microscopy. Images were collected at the speed of 20 frames second -1 with an exposure time of 142.7ms, for a total of 250 frames. An exemplary optical frame of ∼ 500 nm in the Z-dimension was chosen and acquired by confocal spinning disk microscopy (maximum intensity projection) (Dual Spinning Disk Confocal Microscope-Revolution, Andor Technologies). In this case the microscopy focus was located in correspondence of the center of a labeled INS-1 cell. Analyses concerning number of granules, density, speed, mean square displacement, etc. of the distinct hIns-SNAP containing SGs is in progress.

### Example 7. TIRF microscopy

Imaging was performed on living INS-1 cells expressing hIns-SNAP and processed according to the dual-color labeling protocol described in Example 4. Images were collected at the speed of 20 frames second -1 with an exposure time of 100ms, for a total of 1000 frames. Slide 10 shows a frame of - 100 nm in the Z-dimension acquired by total internal reflection microscopy (Olympus IX 71, Andor iXon+ DU 897E, Olympus PLAPO 100x/1.45 TIRFM, Andor IQ version 1.7) in correspondence of the plasma membrane region of a labeled INS-1 cell.
Analyses concerning number of granules, density, speed, mean square displacement, etc. of the distinct hIns-SNAP containing SGs is in progress.

### Example 8. Human insulin specific RIA

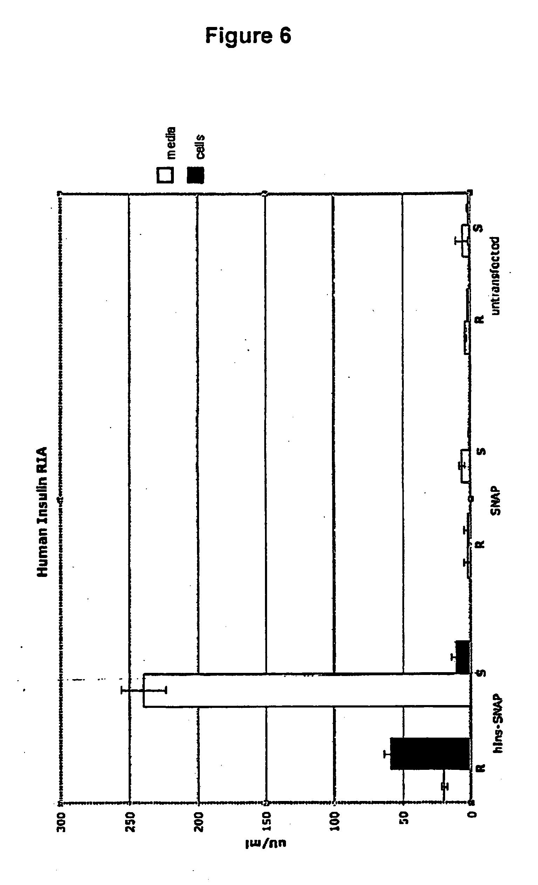

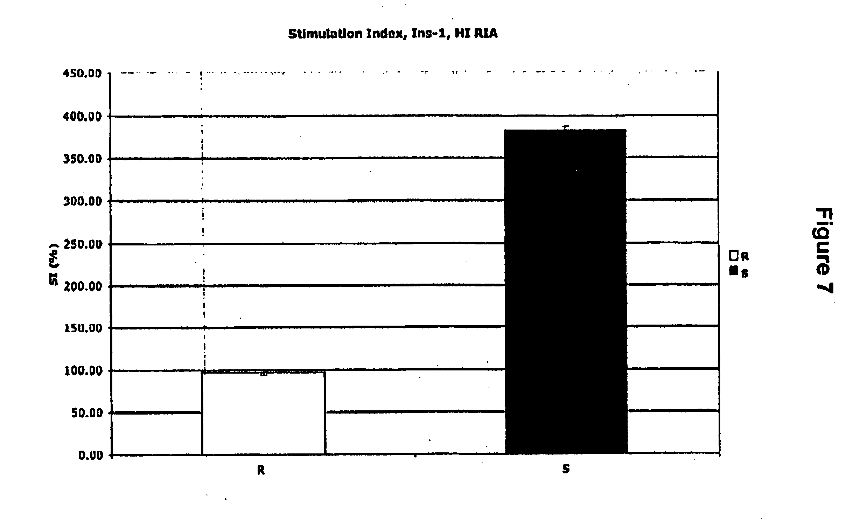

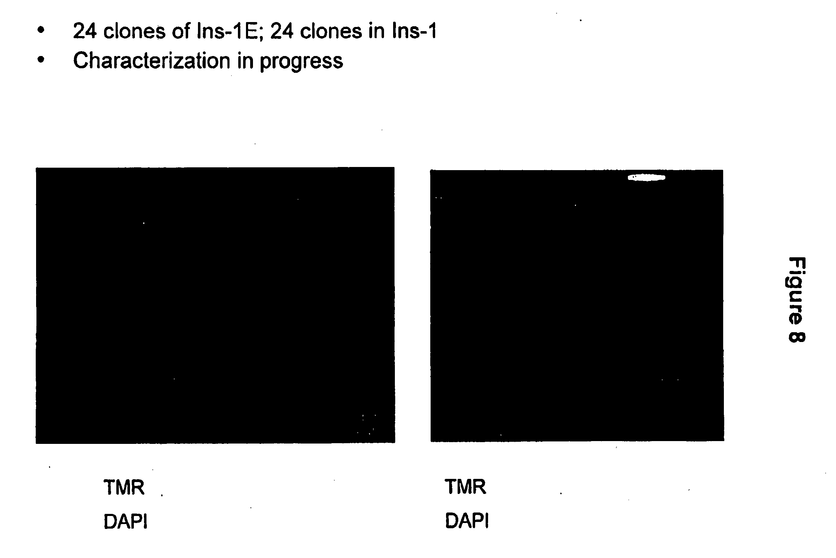

In order to determine whether hINS-SNAP, similarly to endogenous insulin, is secreted in a regulated fashion, the release of hINS-SNAP from INS-1 cells was measured using a radioimmunoassay specific for human insulin (Linco, HI-14K). Cells were transfected with pEG-Ins-SNAP and incubated with resting buffer for 1 hour prior to stimulation. Then the stimulation buffer was applied for 1.5 or 2 hours and the media was collected and frozen. Insulin content was extracted from the cells using an established protocol. RIA measurements showed that human insulin is secreted from the cells upon stimulation (see Figures 6 and 7 and 9).
These results corroborate the glucose-stimulated release of hINS-SNAP from transfected cells. Notably, the RIA that was employed to measure human insulin (Figure 9) only reacts with mature insulin, while it does neither cross-react with human pro-insulin, nor with rat insulin, which is endogenously expressed in INS-1 cells. Accordingly, these data confirm that INS-1 cells properly convert human pro-insulin-SNAP into insulin-SNAP and that the latter is correctly folded. These conclusions are further supported by evidence that insulin (Figure 9) is released in a ratio of 1:1 relative to the human C-peptide (Figure 10).

### Example 9. Measurement of fluorescently labeled hIns-SNAP

hINS-SNAP was labeled with either BG505 or TMR-Star. Figure 11 and Figure 12 show the fluorescence signals detected in the green (emission filter: 535/25 nm) and in the red (emission filter: 590/20 nm) channels, respectively. A) Old and newly generated hINS-SNAP, i.e. old and newly generated secretory granules (SGs) were labeled with TMR-Star (red hINS-SNAP) and BG505 (green hINS-SNAP), respectively, following the same protocol described in the legend of Figure 3. B) and C). Only old SGs were labeled with either BG505 (green hINS-SNAP; B) or TMR-Star (red HINS-SNAP; C), by omitting the incubation with the second fluorophore after the blocking step with BTP. D) and E). Background fluorescence detected in water (D) or in the media of transfected INS-1 cells, expressing HINS-SNAP, but not labelled with a flourophore (E). These new results demonstrate that fluorescently labeled hlns-SNAP can be reliably and conveniently measured by fluorimetry without resorting to the use of more laborious, time-consuming and expensive antibody-based assays, such as RIA and ELISA. Moreover, these data point to the preferential release of newly synthesized HINS-SNAP relatively to the old hINS-SNAP (compare signal levels in A and B in Figure 11).

### Example 10. Localization of hIns-SNAP

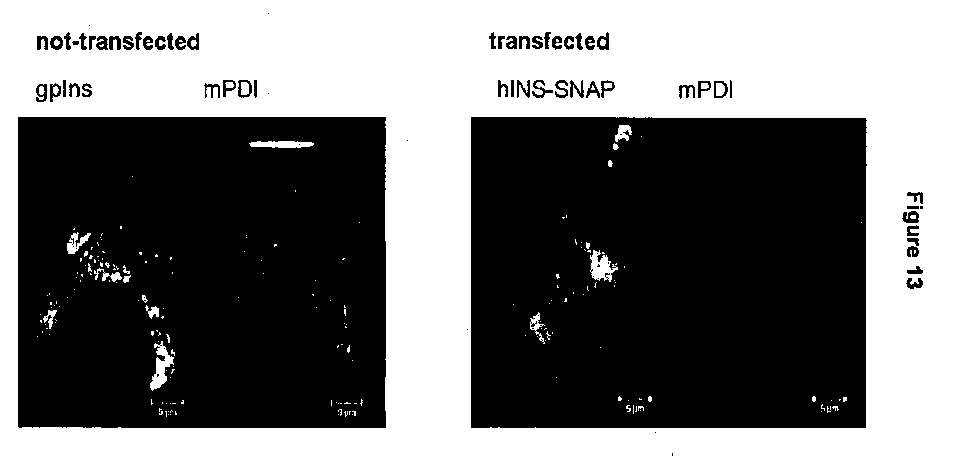

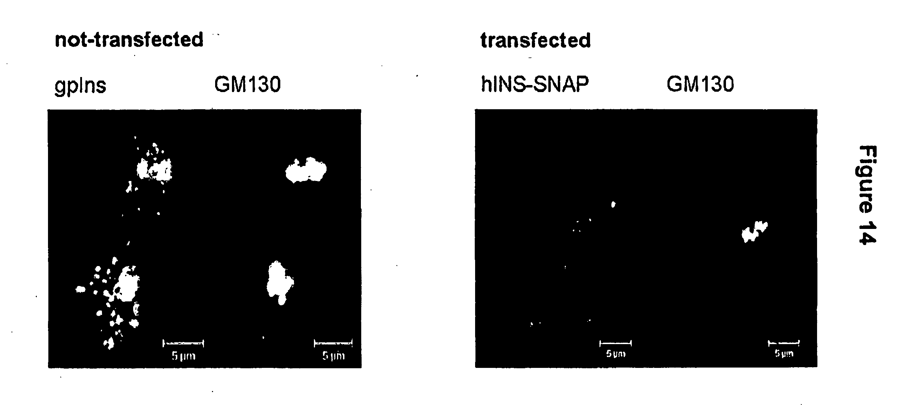

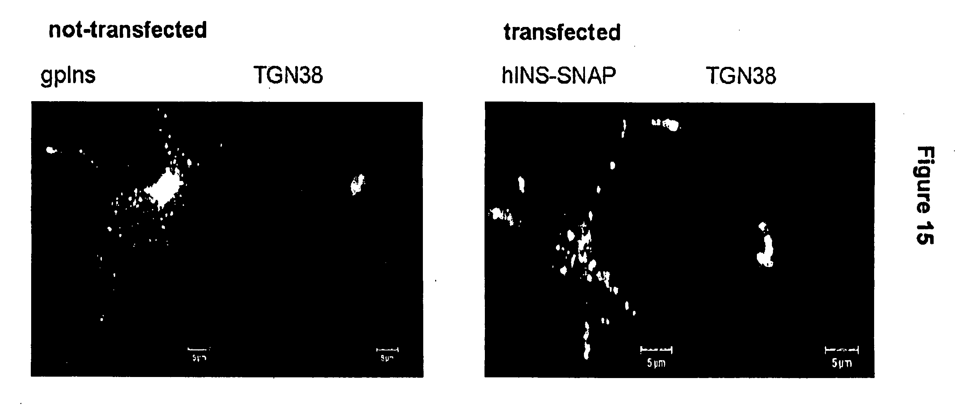

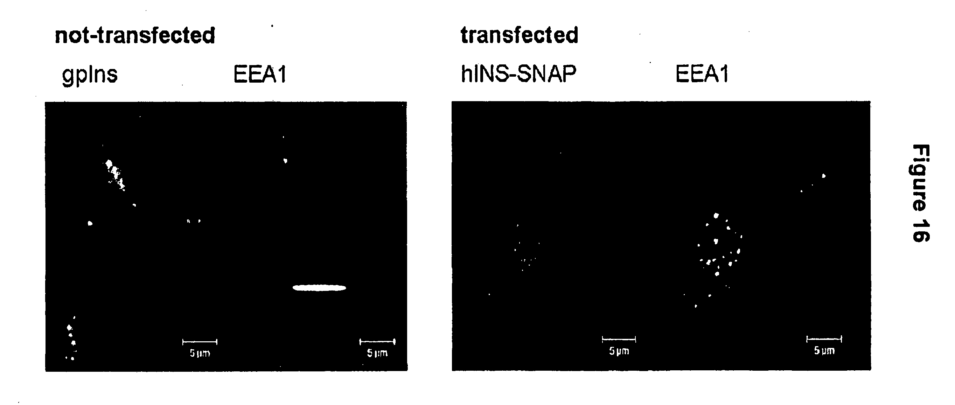

Double fluorescence labelings for insulin and markers of the ER (protein disulfide isomerase, PDI), early cisternae of the Golgi complex (Golgi matrix protein of 130kDa, GM130), trans-Golgi network (TGN38) and early endosome (early endosome antigen-1, EEA1). The distribution of hINS-SNAP in transfected INS-1 cells was visualized by labeling with TMR-Star, while the distribution of endogenous insulin in not-transfected INS-1 cells and of all other proteins was detected using specific primary antibodies followed by Alexa-conjugated secondary antibodies. These results indicate that hINS-SNAP, similar to endogenous rat insulin, is not enriched in the ER (Figure 13) and in early endosomes (Figure 16), while it is detectable in the Golgi compartment (Figures 14 and 15).
The images were acquired using a Zeiss Axiovert 200M confocal microscope and Zeiss LSM 510 AIM Version 4.0 software (Zeiss Plan-Apochromat 63x/1.4 Oil, Zeiss LSM 510 Scanhead with Photomultiplier Tubes, red channel: LP575, green channel: BP 505-550, for the fluorophore combinations, where bleed-through was detected from the red into the green channel, the more narrow filter BP 505-530 was used).

### Example 11. Co-migration of the SG markers CPE and ICA512 together with hINS-SNAP in fractions containing isolated SGs and comparison with early Golgi cisternae marker GM130.

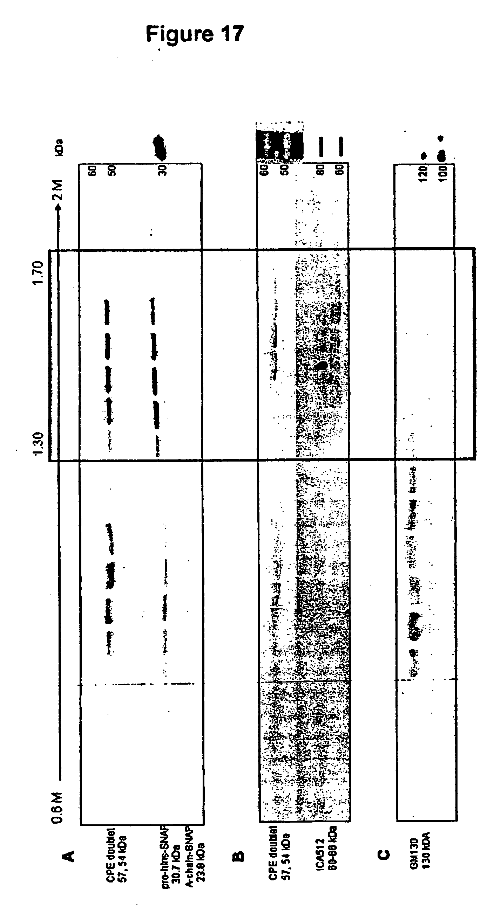

Western blottings on subcellular fractions of hIns-SNAP-transfected INS-1 cells were carried out. Post-nuclear supernatants of hIns-SNAP-transfected INS-1 cells were separated on 0.6-2.0 M continuous sucrose density gradients. Both pro-hINS-SNAP and A-Chain SNAP were detected at the expected molecular weight of -30.7 kD and ∼23.8kD and were enriched in the fractions with 1.3-1.7 M sucrose, where mature insulin SGs are found, as indicated by the enrichment of the fastest (processed) species of CPE (Figure 17A) and ICA512 (Figure 17B). A second peak of pro-hINS-SNAP was detected in lighter fractions (Figure 17A), where Golgi cisternae are found, as indicated by the enrichment for GM130 (Figure 17C).

### References

Alarcón, C., Lincoln, B., Rhodes, C.J.: The biosynthesis of the subtilisin-related proprotein convertase PC3, but no that of the PC2 convertase, is regulated by glucose in parallel to proinsulin biosynthesis in rat pancreatic islets. J Biol Chem. 1993, 268(6):4276-80.
Borgonovo, B., Ouwendijk, J., Solimena, M.: Biogenesis of secretory granules. Curr Opin Cell Biol. 2006, 18(4):365-70.
Bebbington, C.R., Renner, G., Thomson, S., King, D., Abrams, D., Yarranton, G.T.: High-level expression of a recombinant antibody from myeloma cells using a glutamine synthetase gene as an amplifiable selectable marker. Biotechnology (N Y) 1992, 10:169-75.
Bratanova-Tochkova, T.K., Cheng, H., Daniel, S., Gunawardana, S., Liu, Y.J., Mulvaney-Musa, J., Schermerhorn, T., Straub, S.G., Yajima, H., Sharp, G.W.: Triggering and augmentation mechanisms, granule pools, and biphasic insulin secretion. Diabetes 2002, 51 Suppl 1:S83-90.
Daniels, D.S., Mol, C.D., Arvai, A.S., Kanugula, S., Pegg, A.E., Tainer, J.A.: Active and alkylated human AGT structures: a novel zinc site, inhibitor and extrahelical base binding. EMBO J. 2000, 19(7):1719-30.
Duncan, R.R., Greaves, J., Wiegand, U.K., Matskevich, I., Bodammer, G., Apps, D.K., Shipston, M.J., Chow, R.H.: Functional and spatial segregation of secretory vesicle pools according to vesicle age. Nature 2003, 422:176-180.
Glombik M.M., Gerdes H.H.: Signal-mediated sorting of neuropeptides and prohormones: secretory granule biogenesis revisited. Biochemie 2000, 82:315-326.
Grabner, C.P., Price, S.D., Lysakowski, A., Fox, A.P.: Mouse chromaffin cells have two populations of dense core vesicles. J. Neurophysiol. 2005, 94:2093-2104.
Guest, P.C., Rhodes, C.J., Hutton, J.C.: Regulation of the biosynthesis of insulin-secretory-granule proteins. Co-ordinate translational control is exerted on some, but not all, granule matrix constituents. Biochem J. 1989, 257(2):431-7.
Guest, P.C., Bailyes, E.M., Rutherford, N.G., Hutton, J.C.: Insulin secretory granule biogenesis. Co-ordinate regulation of the biosynthesis of the majority of constituent proteins. Biochem J. 1991, 274 (Pt 1):73-8.
Juillerat, A., Gronemeyer, T., Keppler, A., Gendreizig, S., Pick, H., Vogel, H., Johnsson, K.: Directed evolution of O6-alkylguanine-DNA alkyltransferase for efficient labeling of fusion proteins with small molecules in vivo. Chem Biol. 2003, 10(4):313-7.
Horton, R.M., H.D. Hunt, S.N. Ho, J.K. Pullen, Pease, L.R.:. Engineering hybrid genes without the use of restriction enzymes: gene splicing by overlap extension. Gene 1989, 77:61-8**.**
Keppler, A., Gendreizig, S., Gronemeyer, T., Pick, H., Vogel, H., Johnsson, K.: A general method for the covalent labeling of fusion proteins with small molecules in vivo. Nat Biotechnol. 2003, 21(1):86-9.
Martin, S.K., Carroll, R., Benig, M., Steiner, D.F.: Regulation by glucose of the biosynthesis of PC2, PC3 and proinsulin in (ob/ob) mouse islets of Langerhans. FEBS Lett. 1994, 356(2-3):279-82.
Meldolesi, J., Chieregatti, E., Luisa Malosio, M.: Requirements for the identification of dense.-core granules. Trends. Cell Biol. 2004, 14:13-19.
Michael, D.J., Ritzel, R.A., Haataja, L., Chow, R.H.: Pancreatic beta-cells secrete insulin in fast- and slow-release forms. Diabetes 2006, 55:600-607.
Murphy, G., Cockett, M.I., Ward, R.V., Docherty, A.J.:: Matrix metalloproteinase degradation of elastin, type IV collagen and proteoglycan. A quantitative comparison of the activities of 95 kDa and 72 kDa gelatinases, stromelysins-1 and -2 and punctuated metalloproteinase (PUMP). Biochem J. 1991, 277 (Pt 1):277-9.
Perrais, D., Kleppe, I.C., Taraska, J.W., Almers, W.: Recapture after exocytosis causes differential retention of protein in granules of bovine chromaffin cells. J. Physiol. 2004, 560:413-428.
Rorsman, P., Renström, E.: Insulin granule dynamics in pancreatic beta cells. Diabetologia 2003, 46(8):1029-45.
Speier, S., Nyqvist, D., Köhler, M., Caicedo, A., Leibiger, I.B. and Berggren, P.-O.: Noninvasive high-resolution in vivo imaging of cell biology in the anterior chamber of the mouse eye. Nature Protocols 2008, 3: 1278-1286.
Solimena, M., Gerdes, H.H.: Secretory granules: and the last shall be first. Trends Cell Biol. 2003, 13:399-402.
Steiner, D.F.: The proprotein convertases. Curr. Opin. Chem. Biol. 1998, 2:31-39.
Taraska, J.W., Perrais, D., Ohara-Imaizumi, M., Nagamatsu, S., Almers, W.: Secretory granules are recaptured largely intact after stimulated exocytosis in cultured endocrine cells. Proc. Natl. Acad. Sci. U.S.A. 2003, 100:2070-2075.
Taupenot, L., Harper, K.L., O'Connor, D.T.: The chromogranin-secretogranin family. N. Engl. J. Med. 2003, 348:1134-1149.
Terskikh, A., Fradkov, A., Ermakova, G., Zaraisky, A., Tan, P., Kajava, A.V., Zhao, X., Lukyanov, S., Matz, M., Kim, S., Weissman, I., Siebert, P.: "Fluorescent timer": protein that changes color with time. Science 2000, 290(5496):1585-8.
Tsuboi, T., McMahon, H.T., Rutter, G.A.: Mechanisms of dense core vesicle recapture following "kiss and run" ("cavicapture") exocytosis in insulin-secreting cells. J. Biol. Chem. 2004, 279:47115-24.
Tsuboi, T., Ravier, M. A., Parton, L. E., Rutter, G. A.: Sustained Exposure to High Glucose Concentrations Modifies Glucose Signaling and the Mechanics of Secretory Vesicle Fusion in Primary Rat Pancreatic β-Cells. Diabetes 2006, 55:1057-1065.

## Claims

1. An in vitro method of detecting secretory granules (SGs) of different ages in a cell by differentially labelling said SGs according to their age comprising:
(a) contacting a cell capable of forming SGs and expressing
a fusion protein comprising
(i) a (poly)peptide specific for SGs and
(ii) a (poly)peptide having a binding site common for at least three different substances A, B and C covalently binding thereto, wherein the (poly)peptide is a SNAP-tag, a HaloTag or an In-Cell Tetracysteine Tag;
wherein said substances are capable of penetrating the cell membrane and wherein at least substances A and C are detectable by different means;
with a substance A targeting said binding site;
(b) saturating said binding sites by contacting the cell with a substance B which blocks the binding sites which were not bound by substance A;
(c) allowing that unbound substance B is removed from the cell or removing unbound substance B from the cell;
(d) contacting the cell with a substance C targeting said binding site on the fusion protein becoming available for binding after step (b); and
(e) detecting for the presence of both detectable substances A and C;
wherein the simultaneous detection of both substances A and C is indicative of the presence of two populations of SGs having a different age.

2. An in vitro method of investigating in a cell the effect of a stimulus on secretory granules (SGs) differentially labelled according to their age comprising:
(a) contacting a cell capable of forming SGs and expressing
a fusion protein comprising
(i) a (poly)peptide specific for SGs and
(ii) a (poly)peptide having a binding site common for at least three different substances A, B and C covalently binding thereto, wherein the (poly)peptide is a SNAP-tag, a HaloTag or an In-Cell Tetracysteine Tag;
wherein said substances are capable of penetrating the cell membrane and wherein at least substances A and C are detectable by different means;
with
a. a substance A targeting said binding site; and saturating the remaining binding sites by contacting the cell with a substance B which blocks the binding sites which were not bound by substance A; or
b. a substance B which blocks said binding site in an amount sufficient to saturate said binding sites
(b) allowing that unbound substance B is removed from the cell or removing unbound substance B from the cell;
(c1) applying a stimulus to the cell;
(d1) contacting the cell with a substance C targeting said binding site on the fusion protein expressed during and/or after step (c); and
(e1) detecting for the presence of substance A and/or C and/or monitoring the number, size, motility, location in the cell, and/or secretion of the differentially labelled SGs obtained in step (a) and (d1); or
(c2) contacting the cell with a substance C targeting the binding site on the fusion protein expressed during and/or after step (c);
(d2) applying a stimulus to the cell; and
(e2) detecting for the presence of substance C and/or monitoring the number, size, motility, location in the cell and/or secretion of the differentially labelled SGs obtained in step (a) and (c2).

3. A non-human transgenic host comprising a polynucleotide encoding a fusion protein comprising
(i) a (poly)peptide specific for SGs, wherein the (poly)peptide specific for SGs is insulin, phogrin, ICA512, carboxipeptidase E/H, chromogranin A, chromogranin B, secretogranin II, protein convertase 1 or 2, amylin, growth hormone, prolactin, ANP, or NPY and
(ii) a (poly)peptide having a binding site common for at least three different substances A, B and C covalently binding thereto, wherein the (poly)peptide is a SNAP-tag, a HaloTag or an In-Cell Tetracysteine Tag.

4. The method of claim 2, wherein the stimulus is a test agent and the effect to be investigated is the ability of the test agent to induce the formation of secretory granules or their secretion or both (SGs) in a cell;
wherein the method further comprises step (f) comparing the results obtained in step (e1) or (e2) with those obtained for a reference cell not having been contacted with the test agent but otherwise treated equally;
wherein an amount of substance A and/or C detected in the cell which is different than that in the reference cell is indicative of the ability of the test agent to affect the formation and/or the secretion of SGs in the cell.

5. The method or the non-human transgenic host of any one of claims 1 to 4, wherein the SG is an insulin granule and the (poly)peptide of step (a) (i) is specific for insulin granules.

6. The method or the non-human transgenic host of any one of claims 1 to 5, wherein substance B binding to the SNAP-tag is BTP.

7. The method or the non-human transgenic host of any one of claims 1 to 6, wherein substance A binding to the SNAP-tag is TMR-Star, BG-505, BG-430 or BG-DAF.

8. The method or the non-human transgenic host of claim 7, wherein substance C binding to the SNAP-tag is TMR-Star, BG-505, BG-430 or BG-DAF.

9. The method of any one of claims 2 and 4 to 8, wherein the stimulus is a chemical substance or radiation.

10. The method of any one of claims 1, 2 and 4 to 9, wherein the detection or monitoring is effected by detecting fluorescence emission after excitation at a proper wavelength, chemiluminescence or light absorbance.

11. The method or the non-human transgenic host of any one of claims 1 to 10, wherein the fusion protein is expressed after introducing a nucleic acid encoding said fusion protein into the cell.

12. The method of any one of claims 5 to 11, wherein the (poly)peptide is insulin, phogrin, ICA512, carboxipeptidase E/H, chromogranin A, chromogranin B, secretogranin II, protein convertase 1 or 2, amylin or another SG-specific neuropeptide endocrine hormone such as growth hormone, prolactin, ANP, and NPY.

13. The method of any one of claims 1, 2 and 4 to 12, wherein substance A or C or both comprise 1. a moiety specifically binding the binding site on the fusion protein and 2. a detectable moiety.

14. The method of any one of claims 1 and 5 to 13 further comprising contacting the cell with a substance D targeting said binding site on the fusion protein becoming available for binding after step (d) and after allowing that unbound substance C is removed from the cell or after removing unbound substance C from the cell.

15. A method for detecting a substance secreted from uniformly or differentially labelled SGs according to their age comprising:
(a) separating a cell capable of forming SGs, expressing
a fusion protein comprising
(i) a (poly)peptide specific for SGs; and
(ii) a (poly)peptide having a binding site common for at least three different substances A, B and C covalently binding thereto, wherein the (poly)peptide is a SNAP-tag, a HaloTag or an In-Cell Tetracysteine Tag and wherein said substances are capable of penetrating the cell membrane and wherein at least substances A and C are detectable by different means
and
(I) treated according to the method of any one of claims 1, 2 and 5 to 14; from its culture medium; and
(b) detecting in the medium the presence of any detectable substance applied for labeling the SGs, if any;
wherein the detection of one or more substances in the medium indicates that secretion from SGs has taken place.

16. The method of any one of claims 1, 2, 4 to 14, further comprising contacting the cell to be subjected to step (a) with a substance B which blocks the binding sites on said (poly)peptide already expressed in the cell and allowing that unbound substance B is removed from the cell or removing unbound substance B from the cell.

## Patentansprüche

1. *In* vitro-Verfahren zum Nachweis sekretorischer Granula (SGs) verschiedenen Alters in einer Zelle durch unterschiedliches Markieren der SGs gemäß ihrem Alter, umfassend:
(a) Inkontaktbringen einer Zelle, die SGs bilden kann und die ein Fusionsprotein exprimiert, das umfasst:
(i) ein für SGs spezifisches (Poly)peptid und
(ii) ein (Poly)peptid, das eine gemeinsame Bindestelle für die kovalente Bindung von mindestens drei verschiedenen Substanzen A, B und C hat, wobei das (Poly)peptid ein SNAP-Tag, ein HaloTag oder ein In-Cell-Tetracystein-Tag ist, wobei die Substanzen die Zellmembran durchdringen können und wobei zumindest die Substanzen A und C mittels unterschiedlicher Mittel nachgewiesen werden können;
mit einer Substanz A, die die Bindestelle bindet;
(b) Sättigen der Bindestellen durch Inkontaktbringen der Zelle mit einer Substanz B, die die Bindestellen blockiert, an die keine Substanz A gebunden wurde;
(c) Zulassen, dass ungebundene Substanz B von der Zelle entfernt wird oder Entfernen ungebundener Substanz B von der Zelle;
(d) Inkontaktbringen der Zelle mit einer Substanz C, die die Bindestelle des Fusionsproteins bindet, die nach Schritt (b) verfügbar wird; und
(e) Nachweis des Vorhandenseins der beiden nachweisbaren Substanzen A und C;
wobei der gleichzeitige Nachweis der beiden Substanzen A und C das Vorhandensein zweier Populationen von SGs mit unterschiedlichem Alter anzeigt.

2. In vitro-Verfahren zum Erforschen der Wirkung eines Stimulus in einer Zelle auf sekretorische Granula (SGs), die gemäß ihrem Alter unterschiedlich markiert sind, umfassend:
(a) Inkontaktbringen einer Zelle, die SGs bilden kann und die ein Fusionsprotein exprimiert, das umfasst:
(i) ein für SGs spezifisches (Poly)peptid und
(ii) ein (Poly)peptid, das eine gemeinsame Bindestelle für die kovalente Bindung von mindestens drei verschiedenen Substanzen A, B und C hat, wobei das (Poly)peptid ein SNAP-Tag, ein HaloTag oder ein In-Cell-Tetracystein-Tag ist, wobei die Substanzen die Zellmembran durchdringen können und wobei zumindest die Substanzen A und C mittels unterschiedlicher Mittel nachgewiesen werden können;
mit
a. einer Substanz A, die die Bindestelle bindet; und Sättigen der verbleibenden Bindestellen durch Inkontaktbringen der Zelle mit einer Substanz B, die die Bindestellen blockiert, an die keine Substanz A gebunden wurde; oder
b. einer Substanz B, die die Bindestelle in einer Menge blockiert, die ausreichend ist, um die Bindestellen zu sättigen;
(b) Zulassen, dass ungebundene Substanz B von der Zelle entfernt wird oder Entfernen ungebundener Substanz B von der Zelle;
(c1) Anwenden eines Stimulus auf die Zelle;
(d1) Inkontaktbringen der Zelle mit einer Substanz C, die die Bindestelle des Fusionsproteins bindet, das während und/oder nach Schritt (c) exprimiert wird; und
(e1) Nachweis des Vorhandenseins der Substanz A und/oder C und/oder Überwachen der Anzahl, Größe, Bewegung, Lokalisierung in der Zelle und/oder Sekretion der unterschiedlich markierten, in Schritt (a) und (d1) erhaltenen SGs;
oder
(c2) Inkontaktbringen der Zelle mit einer Substanz C, die die Bindestelle des Fusionsproteins bindet, das während und/oder nach Schritt (c) exprimiert wird; und
(d2) Anwenden eines Stimulus auf die Zelle;
(e2) Nachweis des Vorhandenseins der Substanz C und/oder Überwachen der Anzahl, Größe, Bewegung, Lokalisierung in der Zelle und/oder Sekretion der unterschiedlich markierten, in Schritt (a) und (c2) erhaltenen SGs.

3. Nicht-menschlicher, transgener Wirt umfassend ein Polynukleotid, das ein Fusionsprotein kodiert, das umfasst:
(i) ein für SGs spezifisches (Poly)peptid, wobei das für SGs spezifische (Poly)peptid Insulin, Phogrin, ICA512, Carboxypeptidase E/H, Chromogranin A, Chromogranin B, Secretogranin II, Proteinkonvertase 1 oder 2, Amylin, Wachstumshormon, Prolactin, ANP oder NPY ist, und
(ii) ein (Poly)peptid, das eine gemeinsame Bindestelle für die kovalente Bindung von mindestens drei verschiedenen Substanzen A, B und C hat, wobei das (Poly)peptid ein SNAP-Tag, ein HaloTag oder ein In-Cell-Tetracystein-Tag ist.

4. Verfahren nach Anspruch 2, wobei der Stimulus ein Testwirkstoff ist und die zu untersuchende Wirkung die Fähigkeit des Testwirkstoffs ist, die Bildung sekretorischer Granula, oder deren Sekretion, oder beides, in einer Zelle zu induzieren;
wobei das Verfahren des weiteren Schritt (f) umfasst, nämlich den Vergleich der Ergebnisse aus Schritt (e1) oder (e2) mit denen, die für eine Referenzzelle erhalten wurden, die nicht mit dem Testwirkstoff kontaktiert, aber sonst gleich behandelt wurde;
wobei eine Menge an in der Zelle nachgewiesener Substanz A und/oder C, die unterschiedlich ist zu der in der Referenzzelle, die Fähigkeit des Testwirkstoffs anzeigt, die Bildung und/oder Sekretion von SGs in der Zelle zu beeinflussen.

5. Verfahren oder nicht-menschlicher transgener Wirt nach einem der Ansprüche 1 bis 4, wobei das SG ein Insulin-Granula und das (Poly)peptid gemäß Schritt (a)(i) spezifisch für Insulin-Granula ist.

6. Verfahren oder nicht-menschlicher transgener Wirt nach einem der Ansprüche 1 bis 5, wobei Substanz B, die an den SNAP-Tag bindet, BTP ist.

7. Verfahren oder nicht-menschlicher transgener Wirt nach einem der Ansprüche 1 bis 6, wobei Substanz A, die an den SNAP-Tag bindet, TMR-Star, BG-505, BG-430 oder BG-DAF ist.

8. Verfahren oder nicht-menschlicher transgener Wirt nach Anspruch 7, wobei die Substanz C, die an den SNAP-Tag bindet, TMR-Star, BG-505, BG-430 oder BG-DAF ist.

9. Verfahren nach einem der Ansprüche 2 und 4 bis 8, wobei der Stimulus eine chemische Substanz oder Strahlung ist.

10. Verfahren nach einem der Ansprüche 1, 2 und 4 bis 9, wobei der Nachweis oder das Überwachen durch fluoreszente Emission nach Anregung bei einer geeigneten Wellenlänge, Chemilumineszenz oder Lichtabsorption ausgeführt wird.

11. Verfahren oder nicht-menschlicher transgener Wirt nach einem der Ansprüche 1 bis 10, wobei das Fusionsprotein exprimiert wird nachdem eine Nukleinsäure in die Zelle eingeführt wurde, die das Fusionsprotein kodiert.

12. Verfahren nach einem der Ansprüche 5 bis 11, wobei das (Poly)peptid Insulin, Phogrin, ICA512, Carboxypeptidase E/H, Chromogranin A, Chromogranin B, Secretogranin II, Proteinkonvertase 1 oder 2, Amylin oder ein anderes SGspezifisches endokrines Neuropeptid-Hormon ist, wie zum Beispiel Wachstumshormon, Prolactin, ANP oder NPY.

13. Verfahren nach einem der Ansprüche 1, 2 und 4 bis 12, wobei Substanz A oder C, oder beide, 1. einen Rest, der spezifisch an die Bindestelle des Fusionsproteins bindet und 2. einen nachweisbaren Rest, umfassen.

14. Verfahren nach einem der Ansprüche 1 und 5 bis 13, des Weiteren umfassend Inkontaktbringen der Zelle mit einer Substanz D, die die Bindestelle des Fusionsproteins bindet, die verfügbar wird nach Schritt (d) und nachdem zugelassen wurde, dass ungebundene Substanz C von der Zelle entfernt wurde oder nach dem Entfernen der ungebundenen Substanz C von der Zelle.

15. Verfahren zum Nachweis einer Substanz, die von gleichmäßig oder unterschiedlich gemäß ihrem Alter markierten SGs sezerniert wird, umfassend:
(a) Abtrennen einer Zelle, die SGs bilden kann, die ein Fusionsprotein exprimiert, das umfasst:
(i) ein für SGs spezifisches (Poly)peptid und
(ii) ein (Poly)peptid, das eine gemeinsame Bindestelle für die kovalente Bindung von mindestens drei verschiedenen Substanzen A, B und C hat, wobei das (Poly)peptid ein SNAP-Tag, ein HaloTag oder ein In-Cell-Tetracystein-Tag ist, wobei die Substanzen die Zellmembran durchdringen können und wobei zumindest die Substanzen A und C mittels unterschiedlicher Mittel nachgewiesen werden können;
und
(I) die gemäß dem Verfahren nach einem der Ansprüche 1, 2 und 5 bis 14 behandelt wurde;
von ihrem Zellkulturmedium; und
(b) Nachweis des Vorhandenseins einer nachweisbaren Substanz, falls vorhanden, die für die Markierung der SGs verwendet wurde, im Medium;
wobei der Nachweis einer oder mehrerer Substanzen im Medium anzeigt, dass Sekretion von SGs stattgefunden hat.

16. Verfahren nach einem der Ansprüche 1, 2, 4 bis 14, des Weiteren umfassend das Inkontaktbringen der Zelle, die Schritt (a) unterworfen werden soll, mit einer Substanz B, die die Bindestellen des Fusionsproteins blockiert, das bereits in der Zelle exprimiert ist und Zulassen, dass ungebundene Substanz B von der Zelle entfernt wird oder Entfernen der ungebundenen Substanz B von der Zelle.

## Revendications

1. Méthode *in vitro* de détection de granules sécrétoires (GS) d'âges différents dans une cellule par un marquage différentiel desdits GS selon leur âge comprenant :
(a) la mise en contact d'une cellule capable de former des GS et d'exprimer une protéine de fusion comprenant
(i) un (poly)peptide spécifique des GS et
(ii) un (poly)peptide comportant un site de liaison commun pour au moins trois substances différentes A, B et C s'y liant de manière covalente, où le (poly)peptide est un marqueur SNAP, un marqueur Halo ou un marqueur de tétracystéine intracellulaire ;
où lesdites substances sont capables de pénétrer la membrane cellulaire et où au moins les substances A et C sont détectables par des moyens différents ; avec une substance A ciblant ledit site de liaison ;
(b) la saturation desdits sites de liaison par la mise en contact de la cellule avec une substance B qui bloque les sites de liaison qui n'ont pas été liés par la substance A ;
(c) une étape permettant que la substance B non liée soit éliminée de la cellule ou l'élimination de la substance B non liée de la cellule ;
(d) la mise en contact de la cellule avec une substance C ciblant ledit site de liaison sur la protéine de fusion devenant disponible pour une liaison après l'étape (b) ; et
(e) la détection de la présence des deux substances détectables A et C ;
où la détection simultanée des deux substances A et C est indicatrice de la présence de deux populations de GS ayant un âge différent.

2. Méthode *in vitro* de recherche dans une cellule de l'effet d'un stimulus sur les granules sécrétoires (GS) marqués de manière différentielle selon leur âge comprenant :
(a) la mise en contact d'une cellule capable de former des GS et d'exprimer une protéine de fusion comprenant
(i) un (poly)peptide spécifique des GS et
(ii) un (poly)peptide comportant un site de liaison commun pour au moins trois substances différentes A, B et C s'y liant de manière covalente, où le (poly)peptide est un marqueur SNAP, un marqueur Halo ou un marqueur de tétracystéine intracellulaire ;
où lesdites substances sont capables de pénétrer la membrane cellulaire et où au moins les substances A et C sont détectables par des moyens différents ;
avec
a. une substance A ciblant ledit site de liaison ; et la saturation des sites de liaison restants par la mise en contact de la cellule avec une substance B qui bloque les sites de liaison qui n'ont pas été liés par la substance A ; ou
b. une substance B qui bloque ledit site de liaison dans une quantité suffisante pour saturer lesdits sites de liaison
(b) une étape permettant que la substance B non liée soit éliminée de la cellule ou l'élimination de la substance B non liée de la cellule ;
(c1) l'application d'un stimulus à la cellule ;
(d1) la mise en contact de la cellule avec une substance C ciblant ledit site de liaison sur la protéine de fusion exprimée durant et/ou après l'étape (c) ; et
(e1) la détection de la présence de la substance A et/ou C et/ou le contrôle du nombre, de la taille, de la motilité, de la localisation dans la cellule, et/ou de la sécrétion des GS marqués de manière différentielle obtenus dans l'étape (a) et (d1) ; ou
(c2) la mise en contact de la cellule avec une substance C ciblant le site de liaison sur la protéine de fusion exprimée durant et/ou après l'étape (c) ;
(d2) l'application d'un stimulus à la cellule ; et
(e2) la détection de la présence de la substance C et/ou le contrôle du nombre, de la taille, de la motilité, de la localisation dans la cellule, et/ou de la sécrétion des GS marqués de manière différentielle obtenus dans l'étape (a) et (c2).

3. Hôte transgénique non humain comprenant un polynucléotide codant pour une protéine de fusion comprenant
(i) un (poly)peptide spécifique des GS, où le (poly)peptide spécifique des GS est l'insuline, la phogrine, ICA512, la carboxypeptidase E/H, la chromogranine A, la chromogranine B, la sécrétogranine II, la protéine convertase 1 ou 2, l'amyline, l'hormone de croissance, la prolactine, le PNA ou le NPY et
(ii) un (poly)peptide comportant un site de liaison commun pour au moins trois substances différentes A, B et C s'y liant de manière covalente, où le (poly)peptide est un marqueur SNAP, un marqueur Halo ou un marqueur de tétracystéine intracellulaire.

4. Méthode selon la revendication 2, dans laquelle le stimulus est un agent à tester et l'effet à rechercher est la capacité de l'agent à tester à induire la formation de granules sécrétoires (GS) ou leur sécrétion ou les deux dans une cellule ;
où la méthode comprend en outre l'étape (f) comparant les résultats obtenus dans l'étape (e1) ou (e2) avec ceux obtenus pour une cellule de référence n'ayant pas été mise en contact avec l'agent à tester mais traitée autrement de manière égale ;
où une quantité de substance A et/ou C détectée dans la cellule qui est différente de celle dans la cellule de référence indique la capacité de l'agent à tester à affecter la formation et/ou la sécrétion des GS dans la cellule.

5. Méthode ou hôte transgénique non humain selon l'une quelconque des revendications 1 à 4, où le GS est un granule d'insuline et le (poly)peptide de l'étape (a) (i) est spécifique des granules d'insuline.

6. Méthode ou hôte transgénique non humain selon l'une quelconque des revendications 1 à 5, où la substance B se liant au marqueur SNAP est la BTP.

7. Méthode ou hôte transgénique non humain selon l'une quelconque des revendications 1 à 6, où la substance A se liant au marqueur SNAP est TMR-Star, BG-505, BG-430 ou BG-DAF.

8. Méthode ou hôte transgénique non humain selon la revendication 7, où la substance C se liant au marqueur SNAP est TMR-Star, BG-505, BG-430 ou BG-DAF.

9. Méthode selon l'une quelconque des revendications 2 et 4 à 8, dans laquelle le stimulus est une substance chimique ou une radiation.

10. Méthode selon l'une quelconque des revendications 1, 2 et 4 à 9, dans laquelle la détection ou le contrôle est effectué par détection d'émission de fluorescence après excitation à une longueur d'onde correcte, de chimioluminescence ou d'absorbance de lumière.

11. Méthode ou hôte transgénique non humain selon l'une quelconque des revendications 1 à 10, où la protéine de fusion est exprimée après introduction d'un acide nucléique codant pour ladite protéine de fusion dans la cellule.

12. Méthode selon l'une quelconque des revendications 5 à 11, dans laquelle le (poly)peptide est l'insuline, la phogrine, ICA512, la carboxypeptidase E/H, la chromogranine A, la chromogranine B, la sécrétogranine II, la protéine convertase 1 ou 2, l'amyline ou une autre hormone endocrinienne neuropeptidique spécifique des GS telle que l'hormone de croissance, la prolactine, le PNA ou le NPY.

13. Méthode selon l'une quelconque des revendications 1, 2 et 4 à 12, dans laquelle la substance A ou C ou les deux comprennent 1. un radical liant spécifiquement le site de liaison sur la protéine de fusion et 2. un radical détectable.

14. Méthode selon l'une quelconque des revendications 1 et 5 à 13, comprenant en outre la mise en contact de la cellule avec une substance D ciblant ledit site de liaison sur la protéine de fusion devenant disponible pour une liaison après l'étape (d) et après une étape permettant d'éliminer la substance C non liée de la cellule ou après l'élimination de la substance C non liée de la cellule.

15. Méthode de détection d'une substance sécrétée à partir de GS marquée de manière uniforme ou différentielle selon leur âge comprenant :
(a) la séparation d'une cellule capable de former des GS, exprimant une protéine de fusion comprenant
(i) un (poly)peptide spécifique des GS ; et
(ii) un (poly)peptide comportant un site de liaison commun pour au moins trois substances différentes A, B et C s'y liant de manière covalente, où le (poly)peptide est un marqueur SNAP, un marqueur Halo ou un marqueur de tétracystéine intracellulaire et où lesdites substances sont capables de pénétrer la membrane cellulaire et où au moins les substances A et C sont détectables par des moyens différents
et
(I) traitée selon la méthode de l'une quelconque des revendications 1, 2 et 5 à 14;
de son milieu de culture ; et
(b) la détection dans le milieu de la présence de toute substance détectable appliquée pour le marquage des GS, si présente ;
où la détection d'une ou de plusieurs substances dans le milieu indique qu'une sécrétion à partir des GS a pris place.

16. Méthode selon l'une quelconque des revendications 1, 2, 4 à 14, comprenant en outre la mise en contact de la cellule à soumettre à l'étape (a) avec une substance B qui bloque les sites de liaison sur ledit (poly)peptide déjà exprimé dans la cellule et une étape permettant que la substance B non liée soit éliminée de la cellule ou l'élimination de la substance B non liée de la cellule.
